# EUROPEAN PATENT SPECIFICATION

(11) **EP 3 058 546 B1**
(45) Date of publication and mention of the grant of the patent: **26.07.2023**
(21) Application number: 14869877.2
(22) Date of filing: 08.10.2014
(51) Int. Cl.: G16H 40/63, G16H 20/30, G16H 40/67

(54) **INFORMATION SHARING METHOD AND DEVICE**
VERFAHREN UND VORRICHTUNG ZUM TEILEN VON INFORMATIONEN
PROCÉDÉ ET DISPOSITIF DE PARTAGE D'INFORMATIONS

(30) Priority: 11.12.2013 CN 201310676238
(43) Date of publication of application: 24.08.2016
(73) Proprietor: Tencent Technology (Shenzhen) Company Limited, Shenzhen, Guangdong, 518057 (CN)
(72) Inventor: LIU, Lejun, Shenzhen Guangdong 518044 (CN); FAN, Liangliang, Shenzhen Guangdong 518044 (CN); LIU, Kai, Shenzhen Guangdong 518044 (CN); LIN, Xiangyao, Shenzhen Guangdong 518044 (CN); SHAN, Yi, Shenzhen Guangdong 518044 (CN); ZHU, Yaxuan, Guangdong 518044 (CN); HE, Qing, Shenzhen Guangdong 518044 (CN); WENG, Leteng, Shenzhen Guangdong 518044 (CN)
(74) Representative: V.O.
(86) International application number: PCT/CN2014/088127
(87) International publication number: WO 2015/085821

(56) References cited:
- WO-A1-2011/152934
- CN-A- 101 980 269
- CN-A- 103 119 620
- US-A1- 2011 098 156
- Christian Schlup: "Automatic Game Matching", , 13 December 2001 (2001-12-13), XP055300150, Retrieved from the Internet: URL:http://dcg.ethz.ch/theses/ws0203/Onlin eMatching_abstract.pdf [retrieved on 2016-09-06]

## Description

### FIELD OF THE TECHNOLOGY

The present disclosure generally relates to communications technology, and in particular, to information sharing methods and devices.

### BACKGROUND OF THE TECHNOLOGY

Devices such as pedometers and treadmills may have functions such as recording exercising/health data. However, such data is usually available only to the user. If the data can be shared with friends, the user can be more motivated to participate in the exercise and/or sport. Some of the smart devices may include software that can transfer the exercise information collected by the device to the cloud servers. Instant communication software may be able to acquire the exercise information from the cloud server and share such information in friend circles. However, such existing technology is complex and hard to implement, resulting in wasting of running time, electricity, and the user's time.

US20110098156, on which the two-part format of claim 1 is based, discloses accessing personalized fitness services through an integrated application available to a portable electronic device. The integrated application can provide a full fitness center experience by introducing potential new customer to a fitness center and then motivating them to return to the fitness center as active members. For example, the integrated application can provide functions to introduce new customers to a fitness center, can provide functions to motivate customers to join and actively visit the fitness center, can provide in-gym motivation, and can provide post-workout motivation.

### SUMMARY

The above deficiencies and other problems associated with the existing technology are addressed by the technology disclosed below. In some embodiments, the technology is implemented in a computer system that has one or more processors, memory and one or more modules, programs or sets of instructions stored in the memory for performing multiple functions. Instructions for performing these functions may be included in a computer program product configured for execution by one or more processors.

According to one aspect of the present invention, there is provided a method of information sharing as defined in claim 1.

According to another aspect of the present invention, the present invention provides a server as defined in claim 10.

Another aspect of the technology involves a non-transitory computer readable storage medium having stored therein instructions, which when executed by a server cause the server to perform the methods described herein.

Some embodiments may be implemented on either the terminal side or the server side of a terminal-server network environment.

### BRIEF DESCRIPTION OF THE DRAWINGS

The aforementioned features and advantages of the technology as well as additional features and advantages thereof will be more clearly understood hereinafter as a result of a detailed description of preferred embodiments when taken in conjunction with the drawings.
FIG. 1 is a flow chart of an information sharing method in accordance with some embodiments;
FIG. 2 is a flow chart of an information sharing method in accordance with some embodiments;
FIG. 3 is a flow chart of an information sharing method in accordance with some embodiments;
FIG. 4 is a flowchart illustrative of an information sharing method in accordance with some embodiments;
FIG. 5 is a flowchart illustrative of an information sharing method in accordance with some embodiments, showing the process of sharing information related to a real-time competition;
FIG. 6 is a flow chat illustrating how a server, a first user terminal, and a health monitoring device interact in accordance with some embodiments;
FIG. 7 is a block diagram of an information sharing apparatus in accordance with some embodiments;
FIG. 8 is a block diagram of an information sharing apparatus in accordance with some embodiments;
FIG. 9 is a block diagram illustrative of modules in a server in accordance with some embodiments;
FIG. 10 is a block diagram illustrative of modules in a server in accordance with some embodiments;
FIG. 11 is a schematic diagram illustrative of a server in accordance with some embodiments.
FIG. 12 is a schematic diagram illustrative of a first client terminal in accordance with some embodiments.

Like reference numerals refer to corresponding parts throughout the several views of the drawings.

### DESCRIPTION OF EMBODIMENTS

Reference will now be made in detail to embodiments, examples of which are illustrated in the accompanying drawings. In the following detailed description, numerous specific details are set forth in order to provide a thorough understanding of the subject matter presented herein. But it will be apparent to one skilled in the art that the subject matter may be practiced without these specific details. In other instances, well-known methods, procedures, components, and circuits have not been described in detail so as not to unnecessarily obscure aspects of the embodiments.

It should be noted that the terms "first", "second", and the like in the present application may be used for describing various elements and components, but are not intended to limit the elements and components. The terms are merely used for distinguishing one element from another element. For example, a first user terminal may be called a first user terminal in one embodiment but a second user terminal in another embodiment. Similarly, the second user terminal may be called a first user terminal in some embodiments. In the same embodiment, the first user terminal and the second user terminal refer to different user terminals.

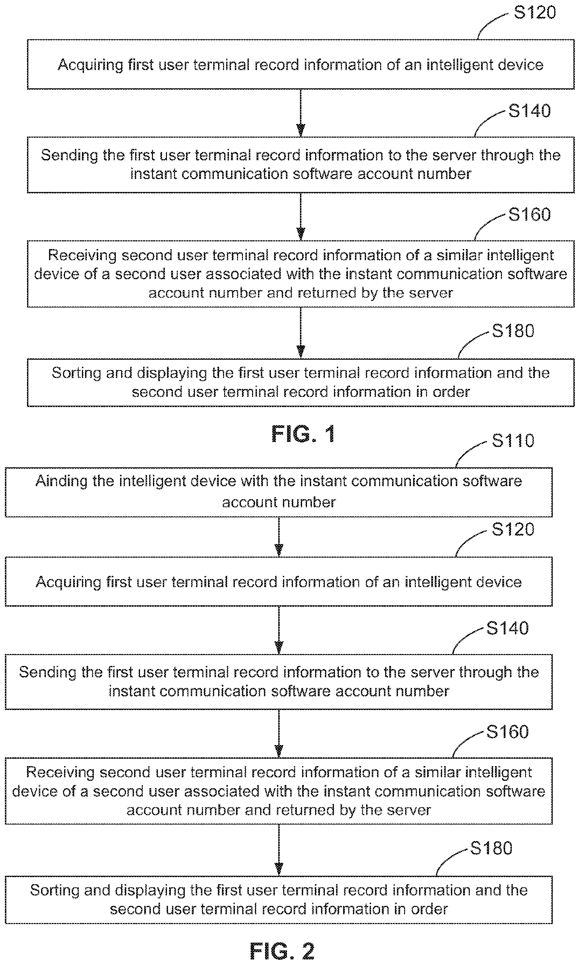

FIG. 1 is a flow chart of an information sharing method according to one embodiment. The embodiment may be specifically applicable to such terminals as a smartphone, a tablet computer, and the like, to acquire the information recorded by an intelligent device such as a pedometer and the like, share the information with the users in a circle of friends through an instant communication software account number, and sort and display the record information of different users. As shown in FIG. 1, the information sharing method of the embodiment comprises the specific steps as follows.

S 120: Acquiring first user terminal record information of an intelligent device. Wherein, the record information of multiple intelligent devices may be acquired, and the intelligent devices are communicated with the first user terminal by using the same protocol. The intelligent devices comprise sports equipment, entertainment equipment and physiological index inspection equipment. Almost all the intelligent devices having a recording function may share and rank the information through the information sharing method of the embodiment.

The sports equipment is one or combinations of a pedometer, a bicycle, a treadmill, a skipping rope, an ergometer, a dart and a racquet. When the information sharing method is used for the sports equipment, the enthusiasm for exercise of the user can be improved, and the user may have a more healthy life-style in entertainment. The entertainment equipment is one or combinations of a game machine, a table tennis and a mahjong machine. Generally games that can be ranked are all in a computer or in a mobile phone. The information sharing method according to the embodiment may rank a real game in a virtual network, which enhances the relevance between the users. The physiological index inspection equipment is one or combinations of a heart beat watch, an electronic blood pressure gauge, a heartbeat detector, an electronic blood sugar detector, an electronic balance scale and a sleep instrument. The recorded information that can be used for sharing and ranking not only refers to the score of sports and entertainment, but also refers to physiological indexes. Medical care personnel may closely monitor the physiological indexes of the user through the shared and ranked record information. For example, a sleeplessness sufferer may use the sleep instrument to share the sleep quality. A diabetes mellitus sufferer may use the electronic blood sugar detector to share the blood sugar index. The medical care personnel may adjust the drug of the sufferer or remind the sufferer for further consultation according to the record information. In one embodiment, friends losing fat together may use the electronic balance scale to share the fat losing effect, and enhance the motivation to lose fat. In another embodiment, multiple intelligent devices may be used at the same time. For example, the travelled distance of bicycle may be shared and ranked; meanwhile, the heart beat frequency recorded by the heart beat watch and the blood pressure may be shared and ranked.

The manner of acquiring the first user terminal record information of the intelligent device comprises one or a combination of acquisition through bluetooth and acquisition through wireless near field communication technology NFC. Bluetooth is a wireless technology supporting short distance communication of the device as long as the intelligent device and a terminal for acquiring information are in a certain distance range, which is convenient for use, and supports to update the first user terminal record information in real time. The wireless near field communication technology NFC requires that the intelligent device and the terminal for acquiring the information are in a closer distance during information interaction, wherein NFC is cheap and can save the cost.

S140: Sending the first user terminal record information to the server (side) through the instant communication software account number. In the embodiment, the first user terminal record information is shared by utilizing a relationship chain of the user in the instant communication software, wherein the instant communication software is a manner that is broadly applied for connecting the relationship between people. Moreover, the instant communication software is flexible to apply, and may be used for sharing and ranking the record information.

S 160: Receiving second user terminal record information of a similar intelligent device of a second user associated with the instant communication software account number and returned by the server. The manner of associating the instant communication software account number with the second user may be set according to the demands of the users, for example, the friends in the instant communication, the users within a certain range determined by the instant communication software, and friends in a group having the same interests and hobbies, and the like.

In one embodiment, a plurality of second users are included. The step 160 may be the step of receiving a predetermined number of top-ranked second user terminal record information among the second user terminal record information of the similar intelligent device of the second user associated with the instant communication software account number and returned by the server. In the embodiment, the second user terminal record information is firstly screened, and only the second user terminal record information ranked in the top five, top ten or the like of second user terminal record information are received, so that the network bandwidth is saved.

S 180: Sorting and displaying the first user terminal record information and the second user terminal record information in order. Preferably, the embodiment may further comprise the step of receiving the ranking information of the first user terminal record information returned by the server. The first user may know the rank in the relationship chain thereof if not in the top rank of a predetermined quantity. More preferably, the embodiment may comprise the step of receiving a predetermined number of rank-closed second user terminal record information among a plurality of second user terminal record information, which are close to the rank of the first user terminal record information and returned by the server, thus being convenient for the first user to know the second user terminal record information ranked close to the first user, know the difference surrounding thereof, and having an encourage effect on the first user.

Referring to FIG. 2, before step S120, the method further comprises a step S 110 of binding the intelligent device with the instant communication software account number. The manners of binding the intelligent device with the instant communication software account number comprise one or combinations of acquisition through Bluetooth, acquisition through a wireless near field communication technology NFC, acquisition through two-dimensional code scanning, and acquisition through word information or digital information inputting. The Bluetooth manner and the NFC manner may be the same with the manner of acquiring the first user terminal record information of the intelligent device, which are more convenient and faster. The manner of acquisition through two-dimensional code scanning and the manner of acquisition through word information or digital information inputting can save cost as a wireless communication protocol used for binding is not needed.

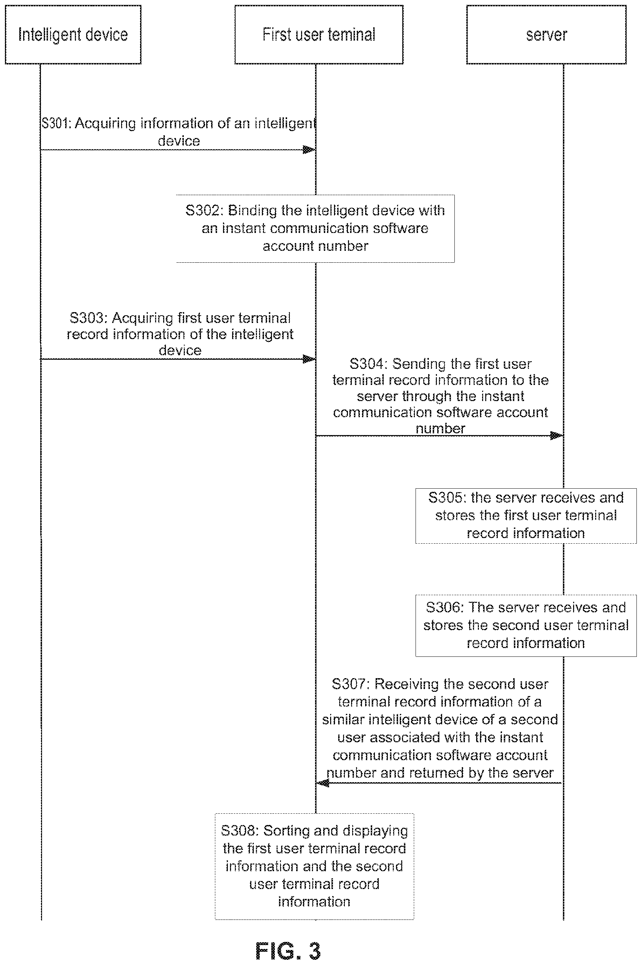

FIG. 3 shows a flow chart of an information sharing method provided in another embodiment of the present application, comprising the steps as follows.

S301: Acquiring information of an intelligent device. A first user terminal may be a mobile phone. The manners for the first user terminal to acquire the information comprise one or combinations of acquisition through Bluetooth, acquisition through a wireless near field communication technology NFC, acquisition through two-dimensional code scanning, and acquisition through word information or digital information inputting. The information is used for binding the intelligent device with the first user terminal. The intelligent device may be such sports equipment as a pedometer, a bicycle, and the like, and may also be such physiological index inspection equipment as a heart beat watch, an electronic blood pressure gauge, and the like.

S302: Binding the intelligent device with an instant communication software account number. After binding, the first user terminal may freely acquire the record information of the intelligent device through the instant communication software.

S303: Acquiring first user terminal record information of the intelligent device. The first user terminal record information refers to the record information generated by a user holding the first user terminal when using the intelligent device. For example, the intelligent device is a pedometer, and the information is acquired through NFC. The user makes the mobile phone close to the pedometer, then the mobile phone acquires the record information such as step frequency accounted by the pedometer, distance, speed, time, and the like, through NFC.

S304: Sending the first user terminal record information to the server through the instant communication software account number.

S305: the server receives and stores the first user terminal record information. The server may send the first user terminal record information to a second user terminal when the second user terminal uploads new second user terminal record information.

S306: The server receives and stores the second user terminal record information. The record information sent by each user is stored on the server, wherein a plurality of second users may be included. The second user terminal record information is stored on the server.

S307: Receiving the second user terminal record information of a similar intelligent device of a second user associated with the instant communication software account number and returned by the server. The first user may receive the second user terminal record information sent by the server. Similarly, the second user may receive the first user terminal record information sent by the server.

S308: Sorting and displaying the first user terminal record information and the second user terminal record information, thus ranking and sharing the information.

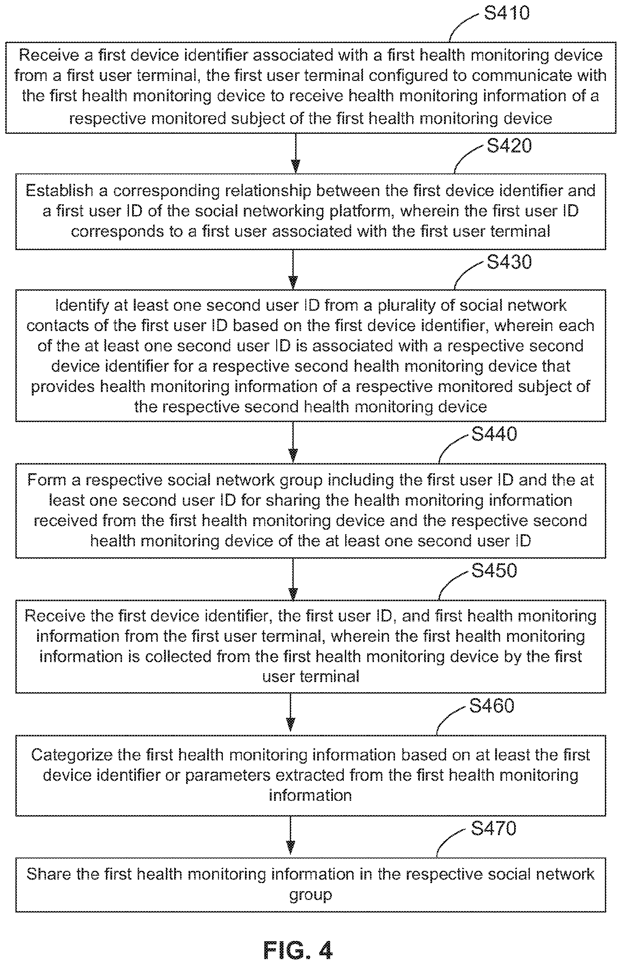

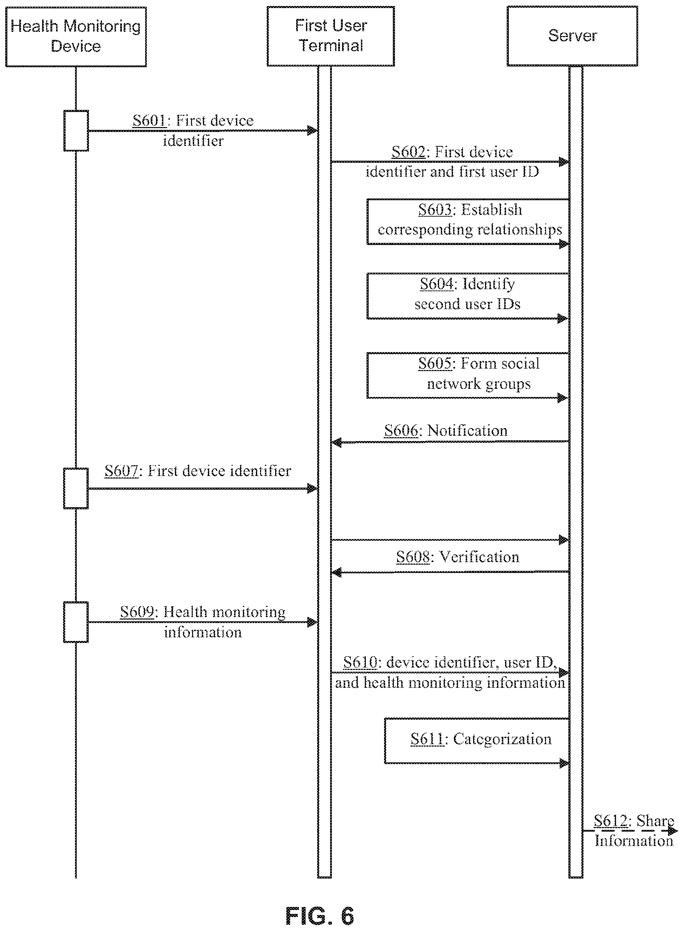

FIG. 4 is a flowchart illustrative of an information sharing method in accordance with some embodiments of the current application. FIG.6 is a schematic illustration of how a server, a first user terminal, and a health monitoring device interact in accordance with some embodiments.

As shown by step S410 of FIG. 4, also referring to step S602 of FIG. 6, the server may receive a first device identifier associated with a first health monitoring device from a first user terminal. In some embodiments, the first user terminal is configured to communicate with the first health monitoring device to receive health monitoring information of a respective monitored subject of the first health monitoring device. As shown by step S601 of FIG. 6, the first user terminal acquires the first device identifier from the first health monitoring device before sending the first device identifier to the server.

The devices herein involved may be any device having basic communication and computational capabilities. For example, the user terminal, e.g. the first user terminal, may be terminals such as but not limited to a desktop computer, a laptop computer, a personal digital assistant (PDA), a tablet computer, a smart phone, an e-book reader, a MP3 (Moving Picture Experts Group Audio Layer III) or MP4 (Moving Picture Experts Group Audio Layer IV) player, a vehicle computer, and a wearable device having computational and communication capabilities. In some embodiments, the user terminal can communicate with other devices wirelessly, e.g. through a network.

The health monitoring device may refer to any device that can monitor, display, record, and/or transfer data related to one or more parameters extracted from information of a user's health and/or exercise or sport activities. Such parameters may include but not be limited to: heart rate, blood pressure, body temperature, respiratory rate, blood sugar level, sleep quality, pedometer value, speed, distance, slope, duration, frequency, weight, resistance, calories, and any combination thereof. In some embodiments, the health monitoring device has data transfer capability, e.g. through a wireless network. In some embodiments, the health monitoring device has data processing activities, e.g. accumulation, recording, categorization and/or ranking. In some embodiments, the health monitoring device can display data. In some embodiments, the health monitoring device can record and store data.

The health monitoring device can be a specifically designed apparatus that monitors health or exercise information. For example, the health monitoring device can be bracelet or wristband or other wearable devices that can monitor, record, and/or transfer parameters such as but not limited to heart rate, blood pressure, body temperature, respiratory rate, blood sugar level, sleep quality, pedometer value, speed, distance, slope, duration, frequency, weight, resistance, calories, and any combination thereof. In some embodiments, the parameters can be collected by health monitoring device such as but not limited to: a heart rate monitoring device (e.g. monitoring watch), an electronic blood pressure gauge, an electronic blood sugar detector, a scale, a sleep monitoring device, and any combination thereof. In some embodiments, the health monitoring device may or may not have other functions. The health monitoring device, e.g. a wristband, can be worn or carried by a user; the health monitoring device, e.g. a sensor and chip, can be embedded or attached to an exercising or sport apparatus, e.g. a treadmill, an elliptical, or a dumbbell.

The health monitoring device can be an exercising or sport apparatus or part of such an apparatus, which is used for exercise and/or sports activities. For example, the health monitoring device can be devices such as but not limited to an abdomenizer, a balance board, a bowflex, a bench-press, a bicycle, a dart, a dumbbell, an elliptical, an ergometer, an exercise ball, an exertris, a fitness trail, gravity boots, grippers, a gymnasticon, a heart rate monitor, a rower, a nordic track, a pedometer, an exercise power tower, a power-plate, a punching bag, a motion ranger, a resistance band, a racquet, a rocker, a roller, a skipping rope, a stair master, a stepping stool, a stationary bicycle, a streetrisder, a thighmaster, a total gym, a treadmill, a stabilizer, a wheelchair trainer, and a yoga mat. The health monitoring device may also be an entertainment device such as but not limited to a game machine, a pool table, or a mahjong machine As indicated, the health monitoring device can also be part of such devices.

In some embodiments, the health monitoring device may comprise or be connected to various sensors such as but not limited to a triaxial accelerometer, a triaxial gyroscope, and a geomagnetic sensor. In some embodiments, the health monitoring device collects the health monitoring information hrough the various sensors.

A device identifier, e.g. the first device identifier, refers to signals, marks, texts, icons, drawings, patterns, codes, numbers, frequencies, or any combination thereof, that can be used to identify a device, e.g. a health monitoring device. For example, the device identifier may be written or recorded in a chip of the health monitoring device. Alternatively, the device identifier may be a code or number that can be displayed by the health monitoring device, wherein the code or number can be scanned or read.

In some embodiments, the health monitoring device has a wireless communication function. For example, the health monitoring device may establish a connection with a user terminal through short distance wireless communication, and then the health monitoring device may send the device identifier to the terminal through the wireless network, so that the terminal may acquire the device identifier of the health monitoring device. Alternatively, the user terminal may trigger the identifier acquiring process by sending a request to the health monitoring device; the health monitoring device can respond to the request based on determination as to whether the user terminal is authorized to access the device identifier; if the user terminal is authenticated and authorized, the health monitoring device sends the device identifier to the user terminal. In some embodiments, the device identifier can be acquired by a user in close proximity to the health monitoring device. For example, the health monitoring device may display a two-dimensional code that encrypts the device identifier, wherein the terminal may acquire the device identifier by scanning and decrypting the two-dimensional code. Alternatively, the user terminal can acquire the device identifier through Bluetooth^{®} or radio frequency identification (RFID) technologies.

The communication program can be any program that assists and facilitates communication between the users. In some embodiments, the communication program is a social networking program. The communication program may have embedded functionalities such as but not limited to: instant messaging, walkie-talkie, audio call, video call, document transfer, conference call, group messaging, bulletin board posting, photographing, videoing, scanning, and audio recording.

In some embodiments, the first user terminal acquires the first device identifier through the communication program. For example, the scanning functionality of the communication program installed on the first user terminal can be used to scan a code, number or icon that encrypts or encodes the first device identifier; or the first device identifier can be sent to the user terminal through the messaging or document transfer functionalities of the communication program. Alternatively, the first user terminal acquires the first device identifier through other programs, wherein the communication program can access and use the first user identifier that has been acquired.

In some embodiments, the first user terminal is configured to control or manage the health monitoring device. For example, the health monitoring device can be a peripheral device of the first user terminal and the health monitoring device is controlled and/or managed by the first user terminal through the communication program. The first user terminal may be equipped with communication interfaces (e.g. bluetooth) and the communication program may have user interfaces (e.g. control buttons to collect data) that are compatible with the health monitoring device.

In some embodiments, the communication program is configured to communicate with the server of a social network platform using a first user ID. The server may be a computer system having computational and networking capabilities. As indicated, the communication program can be a social networking application. The server can manage or operate a social network platform for the communication program, where users exchange information such as messages, audio, picture, and video. The server can be a chat server, an instant messaging server, a microblog server (e.g. Tweeter^{®} or Weibo^{®}), or a blog server. In some embodiments, to log in the server through the communication program, the users use user IDs. Here, the communication program installed on the user terminal is configured to communicate with the server using a first user ID, wherein the first user ID is associated with the user of the user terminal. In some embodiments, the user of the user terminal logs in the communication program with the first user ID. In some embodiments, when the first user terminal acquires the first device identifier, the first user ID has been logged in.

In some embodiments, the communication program is further configured to receive health monitoring information of a respective monitored subject of the first health monitoring device. The monitored subject may refer to a person using the health monitoring device. The monitored subject may be the user of the user terminal, wherein the user is associated with the user ID. The monitored subject may also be a person who is not the user of the user terminal.

The health monitoring information may be information related to health, physical condition, and/or exercise or sport activity of the monitored subject. For example, the health monitoring information may include information related the type of exercise/sport activity such as but not limited to: walking, running, climbing, eclipsing, cycling, weight lifting, stretching, swimming, object throwing, and any combination thereof. The activity may also refer to more complex behavior such as but not limited to: body weight adjustment and sleep training. The types of exercise and/or sport activities are almost limitless. The health monitoring information may also include one or more parameters of a user's health and/or exercise or sport activities. Such parameters may include but not be limited to: heart rate, blood pressure, body temperature, respiratory rate, blood sugar level, sleep quality, body weight, height, body mass index (BMI), pedometer value, speed, distance, slope, duration, frequency, weight, resistance, calories, and any combination thereof.

In some embodiments, the first user terminal may send a request to the server to bind the first device identifier and the first user ID-establishing a corresponding relationship between the first device identifier and first user ID. The first device identifier and the first user ID may be parts of the request or may be sent separately from the request. The request may comprise informational items that allow the server to bind the first device identifier with the first user ID. For example, the request to the server may include informational items such as but not limited to: the first device identifier associated with the first health monitoring device, the first user ID, and a request that the server binds the first device identifier with the first user ID. In some embodiments, the request includes the first device identifier without the first user ID because the server can identify the first user ID based on which device sends out the request. If there is an existing corresponding relationship between the first user terminal and the first user ID, the server can identify the first user ID based on information such as but not limited to a user terminal identifier, wherein the user terminal identifier is sent to the server together with the first device identifier or separately. In some embodiments, the server can establish a corresponding relationship between the first device identifier and the first user ID of the social networking platform

As shown by step S420 of FIG. 4, also referring to step S603 of FIG. 6, the server may establish a corresponding relationship between the first device identifier and a first user ID of the social networking platform, wherein the first user ID corresponds to a first user associated with the first user terminal.

The form of the corresponding relationship between the first device identifier and the first user ID may vary. In some embodiments, the first device identifier and the first user ID uniquely correspond to each other: a search with the first device identifier for user ID will find only the first user ID; and a search with the first user ID for device identifier will find only the first device identifier. In some embodiments, the first device identifier uniquely corresponds to the first user ID: a search with the first device identifier for user ID will find the first user ID and possibly other user IDs that are associated with other users and other user terminals; but a search with the first user ID for device identifier will find only the first device identifier. In this case, the first device identifier, which can be used to identify the first health monitoring device, is possibly associated with more than one user ID and more than one user terminal-the first health monitoring device is used by more than one user in the embodiments of the current method. In some embodiments, the first user ID corresponds to the first device identifier: a search with the first device identifier for user ID will find only the first user ID; but a search with the first user ID for device identifier will find the first device identifier and possibly other device identifiers that are associated with other health monitoring devices. In this case, the user ID, which is associated with the first user terminal and can be used to identify a user of the first user terminal, can be associated with more than one device identifier-the user of the first user ID and the first user terminal can be using more than one health monitoring device. In some embodiments, the first device identifier and the first user ID correspond to each other, but not uniquely: a search with the first device identifier for user ID will find the first user ID and possibly other user IDs; and a search with the first user ID for device identifier will find the first device identifier and possibly other device identifiers.

As shown by step S430 of FIG. 4, also referring to step S604 of FIG. 6, the server can identify at least one second user ID from a plurality of social network contacts of the first user ID based on the first device identifier. In some embodiments, each of the at least one second user ID is associated with a respective second device identifier for a respective second health monitoring device that provides health monitoring information of a respective monitored subject of the respective second health monitoring device.

As shown by step S440 of FIG. 4, also referring to step S605 of FIG. 6, the server can form a respective social network group including the first user ID and the at least one second user ID for sharing the health monitoring information received from the first health monitoring device and the respective second health monitoring device of the at least one second user ID.

In the communication program, a user ID is usually linked with a number of other user IDs, forming a social network structure. Some of the user IDs are directly connected, forming a "direct contact," "good buddy," or "zero degree contact" relationship. In some cases, such direct connections usually allow two user IDs to send and receive messages in a communication directly and not in a group context. In some cases, such direct connections allow two user IDs to view and share certain information through postings, e.g. friend circles. In addition to the direct connections, two user IDs may be connected through other user IDs, making it an indirect connection. Based on the minimum number of interim user IDs, the connections between two user IDs can be categorized based on the "degree" of connection. For example, a zero degree connection refers to the direct connection where two user IDs are connected without any interim user ID; a first degree connection refers to the connections with one interim user ID; a second degree connection refers to the connections with two interim user IDs, etc.

After the server establishes a corresponding relationship between the first device identifier and the first user ID, the server identifies at least one second user ID from a plurality of social network contacts of the first user ID. If the first user ID is not sent to the server, the server can conduct a search based on the first device identifier and find the first user ID; if the first user ID is not uniquely identified the server can ascertain the first user ID based on additional conditions. The second user ID can be from the social network contacts of the first user ID in the communication program. In some embodiments, each of the at least one second user ID is associated with a respective second device identifier for a respective second health monitoring device that provides health monitoring information of a respective monitoring subject of the respective second health monitoring device.

When the server identifies the at least one second user ID, the server can first use different degree of contact. For example, the server can identify all the zero degree contacts of the first user ID and select all the identified contacts as the second user IDs. The server can also identify contacts of the first user ID to a pre-determined and/or adjustable degree, e.g. first degree contacts or second degree contacts. In some embodiments, the plurality of social network contacts of the first user ID consists of all user IDs that are contacts of at least a predetermined degree to the first user ID.

The process of identifying the at least one second user ID based on the first device identifier includes determining a respective device type for the first health monitoring device based on the first device identifier, e.g. the server applies additional conditions to select from the identified contacts. The server selects all the identified contacts that have been associated with at least one device identifier. The server selects all the identified contacts that have been associated with device identifiers of certain types of health monitoring devices. The health monitoring device may be any device that monitors, records, or transfers information related to health, exercise, and/or sports. The health monitoring device may have a device type (e.g. treadmill, eclipse, heart rate monitoring wristband, etc.) that is reflected or encoded by the device identifier. Based on the device identifier, e.g. the first device identifier for the first health monitoring device, the server can determine the device type. For each of the plurality of social network contacts of the first user ID, the user terminal determines whether the social network contact is associated with a respective device identifier for a device of the respective device type, and in accordance with a determination that the social network contact and the first user ID are both associated with the respective device identifiers of the same device type, the user terminal includes the social network contact as one of the at least one second user ID.

In some embodiments, the respective device type is predefined based on a type of activity that is monitored by the first health monitoring device. For example, the device type may be "devices that can monitor running activities" and devices such as treadmills (or the monitoring accessories of treadmills) and pedometers may have the same device type. The identification of the second user ID based on device types, therefore, may allow the identification of user IDs associated with similar functions.

In some embodiments, the respective device type is predefined based on a type of health or exercise data that is provided by the first health monitoring device. For example, devices that can measure the heart rate of the user can be identified to have the same device type. The user IDs associated with device identifiers of these devices may be included in the second user ID for the social network group for sharing the health monitoring information.

In some embodiments, the respective device type is applicable to identify user IDs that are associated with health monitoring devices of different brands, manufacturers, and data format. Therefore, a same device type may be defined by different criteria in different embodiments and there is no need that a same device type means that every aspect of the health monitoring device is the same. For example, both a wristband and a treadmill can monitor heart rate of the user; though the wrist hand and the treadmill have different brands, are from different manufacturers, and use different data format, in some embodiments the wristband and the treadmill can still be considered to have the same device type and the user IDs associated with the wristband and the treadmill can be considered the second user IDs and included in the social network group. Such an approach allows health monitoring devices that have significant differences to be used by the same social network group through the same communication program, providing more compatibility and flexibility.

The first device identifier is associated with at least two device types or at least two exercise data types, and wherein forming the respective social network group including the first user ID and the at least one second user ID further comprises: forming a respective social network group for each of the at least two device types or each of the at least two exercise data types, wherein the respective social network group includes the first user ID and one or more of the at least one second user ID that correspond to the same device type or exercise data type. For example, the first device identifier can be a wristband that has global positioning system (GPS) functions and the wristband can monitor the heart rate of the user and the speed of the user. The server, therefore, can form two social network groups, wherein one group includes the first user ID and respective second user IDs that share heart rate information during exercise, and the other social network group includes the first user ID and respective second user IDs that share speed information during exercises involved activities such as running and cycling. Therefore, it is possible for the server to form more than one social network group for the same first user ID and the respective second user ID to share different health monitoring information.

Besides the degree of contact, association with device identifier, and type of health monitoring device, other and/or addition criteria can also be used by the server in identification of the respective second user IDs and formation of the social network group. For example, the server can identify contacts that are in a geological area (e.g. in a same city as the user of the user terminal) as the second user ID. It should also be noted that the user associated with the first user ID may also be able to configure how the server identifies the second user ID. For example, the user terminal may present a series of choices, selections, and criteria to the user of the user terminal so that the user can partially control the server's identification of the second user ID.

In some embodiments, the server identifies at least one second user ID that form a social network group with the first user ID. If the server cannot identify at least one second user ID, the server can change the identification criteria automatically and/or send a notification to the user terminal to allow the user to adjust the criteria to increase the chance of finding a second user ID.

After identifying the at least one second user ID, the server can form a respective social network group including the first user ID and the at least one second user identifier for sharing the health monitoring information received from the first health monitoring device and the respective second health monitoring device of the at least one second user ID. In some embodiments, such a social network group is formed solely for the sharing of the health monitoring information. In some embodiments, the social network group is formed to share other information as well. In some embodiments, the formation of the social network group may also require individual consent from the second user IDs. In some embodiments, the formation of the social network group does not require addition consent from the second user IDs that have previously consented, e.g. in an agreement to the server when setting up the user ID, to be added to such social network groups automatically.

As shown by step S606 of FIG. 6, after forming the social network group, the server can then send a notification to the first user terminal regarding the social network group. The notification allows the first user terminal to keep a record of what social network group has been formed to share health monitoring information. In some embodiments, the members of the social network group are disclosed in the notification. In some embodiments, the user of the first user terminal is allowed to edit, e.g. deleting, the members of the social network group, at least the members that will receive health monitoring information from the first user terminal. Therefore, in response to sending the request, the user terminal receive a notification from the server regarding a respective social network group for sharing the health monitoring information received from the first health monitoring device with at least one second user ID identified from a plurality of social network contacts of the first user ID.

In some embodiments, after a device identifier is acquired by the first user terminal, a determination needs to be made regarding whether the device identifier has been bound to the first user ID. If the first device identifier is bound to a first device identifier, the first user terminal and/or the server need to further determine whether the acquired device identifier is the first device identifier that has been bound to the first user terminal.

In some embodiments, after acquiring the first device identifier, the user terminal can detect, through the server or only on the user terminal itself, whether the first user ID is bound to the first device identifier. If steps, e.g. steps S602-S605 of FIG. 6, have been taken to establish the corresponding relationship between the first device identifier and the first user ID, the first user terminal and/or the server, with or without communications between the first user terminal and the server, can determine that it is no longer necessary to bind the first device identifier with the first user terminal. Otherwise, the method proceeds to steps S602-S605, where the user terminal sends a request to the server to bind the first device identifier with the first user ID.

As shown by steps S607 and S608 of FIG. 6, after the first user terminal acquires a device identifier and sends the acquired device identifier to the server, the first user terminal and/or the server can determine whether the acquired device identifier is properly associated with the first user ID associated with the first user terminal. In some embodiments, such a determination can be considered a verification and can be made by the server or through communications between the user terminal and the server. In particular, the user terminal and the server can determine whether the first user ID logged in the communication program on the first user terminal matches a saved user ID on the server. For example, after acquiring the first device identifier, the first user terminal can send the first device identifier and the first user ID to the server, wherein the server conducts a search with the first device identifier to identify a saved user ID and compare the saved user ID with the first user ID-the results is positive (successful) when the saved user ID and the user ID from the user terminal are the same. However, it should also be noted that the verification step described for step S608 of FIG. 6 can use alternative approaches. For example, the user terminal and the server can search with the first user ID to find a saved device identifier and compare the saved device identifier with the acquired first device identifier-if the device identifiers match then the verification result is positive (successful).

When the verification is successful, the first user terminal receives first health monitoring information from the first health monitoring device. On the other hand, when the verification is unsuccessful, the first user terminal may refuse to accept first health monitoring information from the first health monitoring device. However, it should also be noted that the user terminal may take different approach to the failure of verification. For example, the user terminal may receive the health monitoring information before the verification is fully conducted; the user terminal may then refuse to process and/or send the health monitoring information to the server if the verification is unsuccessful.

When the verification is successful, the user terminal can receive first health monitoring information from the first health monitoring device. In some embodiments, the health monitoring information is based on monitored activities of a first user associated with the first user terminal. In some embodiments, the first user terminal can receive the first device identifier and the first health monitoring information from the first health monitoring device at the same time. The first user terminal can extract the first device identifier and conduct the verification process by communicating with the server. If the verification is successful, the first user terminal and the server can process the health monitoring information. In some embodiments, the first health monitoring information is received only after the verification is successful.

As shown by step S609 of FIG. 6, the first user terminal receives first health monitoring information from the first health monitoring device. In some embodiments, the health monitoring information is based on monitored activities of a first user associated with the first user terminal.

In some embodiments, the first user, the first user terminal, and the first user ID have unique corresponding relationships between one another. As indicated, "first" does not refer to any particular user, device or identifier, but to user, device, or identifier that is being described to conduct the actions herein provided.

The monitored activities are activities that can be performed on the health monitoring device or devices associated with the health monitoring device. For example, the monitored activity can be walking, running, climbing, eclipsing, cycling, weight lifting, stretching, swimming, object throwing, and any combination thereof. The monitored activity may also refer to more complex behavior such as but not limited to: body weight adjustment and sleep training.

The health monitoring information, e.g. first health monitoring information, is information from a health monitoring device, e.g. first health monitoring device. The health monitoring information can include parameters such as but not be limited to: heart rate, blood pressure, body temperature, respiratory rate, blood sugar level, sleep quality, pedometer value, speed, distance, slope, duration, frequency, weight, resistance, calories, and any combination thereof. In some embodiments, the health monitoring information includes information related to the type of the activity this is being monitored.

In some embodiments, the first health monitoring device monitors activities of the first user associated with the first user terminal, collects first health monitoring information, and sends the first health monitoring information to the first user terminal. The first health monitoring device may or may not filter the information that has been collected. In some embodiments, the user terminal may control what information is collected by the health monitoring device or what information can be considered health monitoring information that is sent to the user terminal.

As shown by step S450 of FIG.4, also referring to step S610 of FIG. 6, the first user terminal sends, and the server receives the first device identifier, the first user ID and the first health monitoring information. In some embodiments, the first health monitoring information is collected from the first health monitoring device by the first user terminal.

As shown by step S460 of FIG. 4, also referring to step S611 of FIG. 6, the server categorizes the first health monitoring information based on at least the first device identifier or parameters extracted from the first health monitoring information.

In some embodiments, the first user terminal processes the health monitoring information before sending the information to the server. For example, the first user terminal may extract only the necessary parameters for a particular social network group from the health monitoring information and send only the parameters to the server. In some embodiments, the first user terminal transfers the first health monitoring information to the server without processing.

In some embodiments, the first user terminal sends both the first device identifier and the first user ID to the server so that the server can verify the corresponding relationship between the first device identifier and the first user ID, as shown by step S608 of FIG. 6. In some embodiments, however, the first user terminal only sends the first device identifier or the first user ID to the server because the corresponding relationship between the first user identifier and the first user ID allows the identification of one item with the other.

The server can categorize the health monitoring information based on different parameters for different embodiments. As indicated, the parameters include but are not limited to: heart rate, blood pressure, body temperature, respiratory rate, blood sugar level, sleep quality, pedometer value, speed, distance, slope, duration, frequency, weight, resistance, calories, and any combination thereof. In some embodiments, the parameters may also include the device type of the health monitoring device. For example, the server can extract the information related to heart rate of the user from the health monitoring information and share the information with a particular social network group. In some embodiments, the server can extract the device type, e.g. treadmill or eclipse, from the health monitoring information and share the health monitoring information between the respective social network groups. For example, the server may share the information of the first user using the treadmill to exercise with the social network group including second user ID associated with treadmill users.

As shown by step S470 of FIG. 4, also referring to step S612 of FIG. 6, the server shares the first health monitoring information in the respective social network group.

The health monitoring information can be shared-transferred from the server to the respective user terminals associated with the second user IDs in the respective social network group. In some embodiments, after categorizing the first health monitoring information based on at least the first device identifier or parameters extracted from the first health monitoring information, the server share the first health monitoring information in the respective social network group. In some embodiments, the sharing of the first health monitoring information is conducted based on a preset schedule, which determines, at least in part, how the health monitoring information is shared between the user IDs in the social network group.

In some embodiments, the preset schedule involves the timing of the sharing and possibly provides other criteria. For example, the user or a default set up can control the preset schedule so that the health monitoring information is shared immediately. In some embodiments, the sharing is immediate and the information is transferred to the user terminals of the second user IDs. In some embodiments, the sharing only takes place when certain conditions are met. For example, the health monitoring information can be transferred to the user terminal of the second user IDs when the health monitoring information indicates that the first user has exercised more than 10 minutes or when the heart rate of the first user has been sustained at a high level, e.g. 150/min, for more than a certain time, e.g. 3 minutes. In some embodiments, the health monitoring information can be shared after a fixed period of time, e.g. 4 hours. In some embodiments, the health monitoring information can be shared after the server has collected health monitoring information from, e.g. at least two members of the social network group. It should also be noted that in some embodiments the preset schedule does not involve the timing of sharing.

The health monitoring information that can be used for sharing and ranking not only refers to the score of sports, exercise, and entertainment, but also to physiological indexes and parameters. Medical care personnel as well as other users, who operate a user terminal, may closely monitor the physiological conditions of the user through the shared and ranked health monitoring information. For example, a sleeplessness sufferer may use the sleep instrument to share sleep quality. A diabetes patient may use the electronic blood sugar detector to share the blood sugar index. The medical care personnel may adjust the drug of the patient or remind the patient for further consultation according to the health monitoring information. In one embodiment, friends losing weight together may use the electronic balance scale to share the weight losing progress, and enhance the motivation to lose weight. In another embodiment, multiple devices may be used at the same time. For example, the travelled distance of bicycle may be shared and ranked among users of a social network group. Meanwhile, the heart rate recorded by the heart rate watch and the blood pressure may be shared and ranked among the users of a social network group.

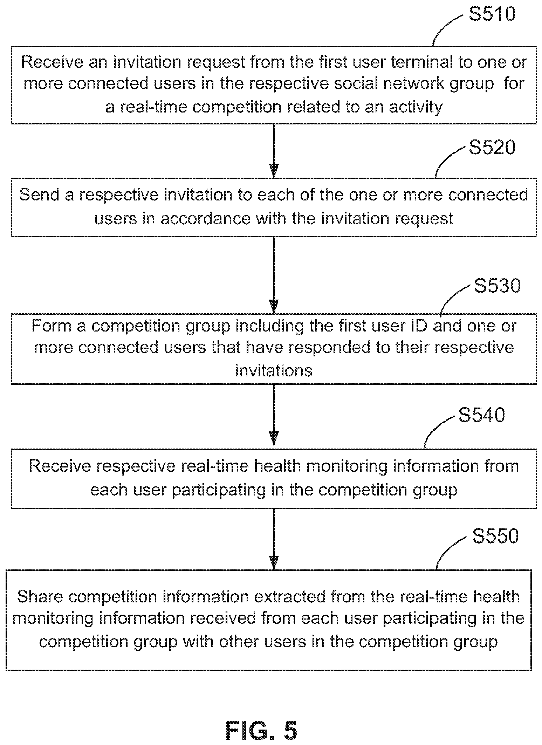

FIG. 5 is a flowchart illustrative of an information sharing method in accordance with some embodiments of the current application, showing the process of sharing information related to a real-time competition.

As shown by step S510 of FIG. 5, the server can receive an invitation request from the first user terminal to one or more connected users in the respective social network group for a real-time competition related to an activity. When a user, e.g. the first user associated with the first user terminal, in the respective social network group intends to participate in an activity, e.g. running, and wants to involve other users in a real-time competition, the user can send out an invitation to other designated users or to a group of users to participate.

In some embodiments, the formation of the social network group and the types of activities allowed to be shared in the social network group enable the users in the group to participate in a real-time competition and effectively share the health monitoring information related to the real-time competition in the group. For example, the social network group is formed based on the activity, e.g. running, being monitored. When the users in the group are running under the monitoring of the health monitoring devices, the collected data-the health monitoring information-can be shared. For a real-time competition, a user, e.g. a first user associated with a first user terminal, a first user ID, a first device identifier, and a first health monitoring device, can send an invitation to the social network group to participate in real-time competition of running. In some embodiments, such an invitation needs to be transferred by the server, which first receives an invitation request from the first user terminal.

As shown by step S520 of FIG. 5, the server can send a respective invitation to each of the one or more connected users in accordance with the invitation request. In some embodiments, the invitation request specifies which users should be contacted regarding joining the real-time competition. In some embodiments, the server determines which users should be contacted, e.g. users in a social network group that is formed by processes described in steps S410-S440 of FIG. 4.

After receiving the invitation, the respective second user terminal presents the invitation at the second user terminal. The second user terminal can implement the technology disclosed herein through the communication program so that the user can determine whether to participate in the real-time competition. In some embodiments, when the second user is engaged in the same activity, it is unnecessary to present the invitation. For example, the second user is already running on a treadmill when an invitation comes in for a real-time competition of running from the first user, it is possible to present the invitation to the second user and it is also possible to omit the steps of seeking permission from the second user and present only a notification that the second user has been entered into the real-time competition of running. This approach may require previous permission from the second user to allow such automatic participation. In some embodiments, the respective second user terminal receives user input accepting the invitation to join the real-time competition. The invitation can be presented in a user interface, where the second user can accept or deny the invitation. In some embodiments, the second user accepts the invitation by making a user input to join the real-time competition.

As shown by step S530 of FIG. 5, the server can form a competition group including the first user ID and one or more connected users that have responded to their respective invitations.

In some embodiments, the respective second user terminal sends a response to the server regarding the acceptance of the invitation. In this regard, the second user terminal informs the server to continue with the connecting process and allow the second user to join the real-time competition. After receiving the notification from the connected users, e.g. the second users, that the user agrees to join the real-time competition, the server forms a competition group including the second user IDs that have responded to the invitation and the first user that sends out the invitation. When more connected users respond to the respective invitations to confirm the intention to join, these users can be added to the competition group in which the health monitoring information related to the real-time competition can be shared.

In some embodiments, the competition group can be formed without invitation from any particular user. For example, when the server detects transmission of real-time health monitoring information from respective user terminals of two or more user IDs in the respective social network group, the server can proactively form a competition group by sending invitations to the users already engaged in the activity. In particular, in response to detecting the transmission, the server can proactively, without request from the respective user terminals of the two or more user IDs in the respective social network group, generates an invitation for the two or more user IDs to participate in a real-time competition based on an activity monitored by the real-time health monitoring information, and sends the invitation to each of the two or more user IDs. When the users respond to the respective invitations to indicate that they agree to join the real-time competition, the server can form the competition group.

As shown by step S540 of FIG. 5, during the real-time competition, the server can receive respective real-time health monitoring information from each user participating in the competition group. The real-time health monitoring information is first received by the user terminals, such as the first user terminal, from the health monitoring device. Then the user terminals transfer the respective real-time health monitoring information to the server with or without processing.

As shown by step S550 of FIG. 5, the server can share competition information extracted from the real-time health monitoring information received from each user participating in the competition group with other users in the competition group. In some embodiments, the server generates a ranking list or provide a comparison (real-time and/or after completion) of the participating users based on parameters extracted from the real-time health monitoring information.

In some embodiments, the server provides real-time comparison of the competition information extracted from the real-time health monitoring information received from each user participating in the competition group to each user participating in the competition group. In some embodiments, the server provides a final comparison of the competition information extracted from the real-time health monitoring information received from each user participating in the competition group to each user participating in the competition group.

For example, the first user terminal receives competition information extracted from real-time health monitoring information gathered from each user participating in the competition by the server. As indicated, the real-time health monitoring information may or may not have been processed by the server. The competition information may include a real-time or after-completion comparison of the competition information extracted from the real-time health monitoring information. In some embodiments, such comparison is presented in the form of a ranking list. By receiving the respective real-time health monitoring information, the first user becomes aware of the progress of the real-time competition.

In some embodiments, the server can limit the competition information sent to the user terminals by listing only the top ranked users, e.g. top 5 or 10 or any preset number. Such an approach may save bandwidth and may motivate the user to improve their performance.

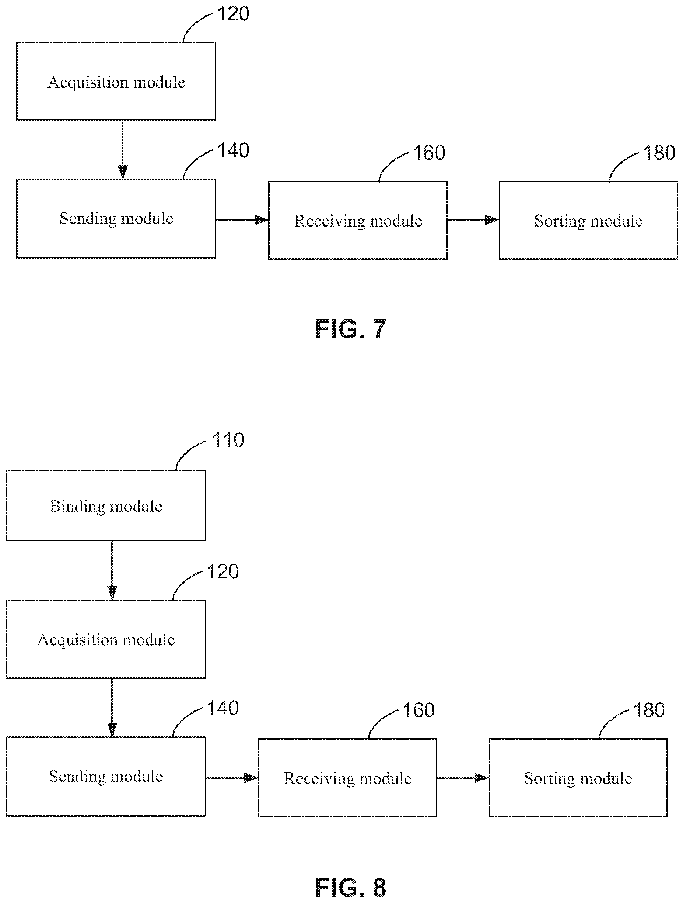

FIG. 7-12 illustrate the computer system and devices that may be used to perform the methods described above. To avoid redundancy, not all the details and variations described for the method are herein included for the devices. Such details and variations should be considered included for the description of the devices as long as they are not in direct contradiction to the specific description provided for the methods.

FIG. 7 is a block diagram of an information sharing apparatus according to one embodiment. The embodiment may be specifically applicable to such terminals as a smartphone, a tablet computer, and the like, to acquire the information recorded by an intelligent device such as a pedometer and the like, share the information with the users in a circle of friends through an instant communication software account number, and sort and display the record information of different users. As shown in FIG. 7, the information sharing apparatus of the embodiment comprises:

An acquisition module 120, used for acquiring first user terminal record information of an intelligent device. Wherein, the record information of multiple intelligent devices may be acquired, and the intelligent devices are communicated with the first user terminal by using the same protocol. The intelligent devices comprise sports equipment, entertainment equipment and physiological index inspection equipment. Almost all the intelligent devices having a recording function may share and rank the information through the information sharing apparatus of the embodiment.

The sports equipment is one or combinations of a pedometer, a bicycle, a treadmill, a skipping rope, an ergometer, a dart and a racquet. When the information sharing apparatus is used for the sports equipment, the enthusiasm for exercise of the user can be improved, and the user may have a healthier life-style in entertainment. The entertainment equipment is one or combinations of a game machine, a table tennis and a mahjong machine. Generally games that can be ranked are all in a computer or in a mobile phone. The information sharing apparatus according to the embodiment may rank a real game in a virtual network, which enhances the relevance between the users. The physiological index inspection equipment is one or combinations of a heart beat watch, an electronic blood pressure gauge, a heartbeat detector, an electronic blood sugar detector, an electronic balance scale and a sleep instrument. The recorded information that can be used for sharing and ranking not only refers to the score of sports and entertainment, but also refers to physiological indexes. Medical care personnel may closely monitor the physiological indexes of the user through the shared and ranked record information. For example, a sleeplessness sufferer may use the sleep instrument to share the sleep quality. A diabetes mellitus sufferer may use the electronic blood sugar detector to share the blood sugar index. The medical care personnel may adjust the drug of the sufferer or remind the sufferer for further consultation according to these record information. In one embodiment, friends losing fat together may use the electronic balance scale to share the fat losing effect, and enhance the motivation to lose fat. In another embodiment, multiple intelligent devices may be used at the same time. For example, the travelled distance of bicycle may be shared and ranked; meanwhile, the heart beat frequency recorded by the heart beat watch and the blood pressure may be shared and ranked.

The manner of acquiring the first user terminal record information of the intelligent device comprises one or a combination of Bluetooth acquisition and wireless near field communication technology NFC. Bluetooth is a wireless technology supporting short distance communication of the device as long as the intelligent device and a terminal for acquiring information are in a certain distance range, which is convenient for use, and supports to update the first user terminal record information in real time. The wireless near field communication technology NFC requires that the intelligent device and the terminal for acquiring the information are in a closer distance during information interaction, wherein NFC is cheap and can save the cost.

A sending module 140, used for sending the first user terminal record information to a server through an instant communication software account number. In the embodiment, the first user terminal record information is shared by utilizing a relationship chain of the user in the instant communication software, wherein the instant communication software is a manner that is broadly applied for connecting the relationship between people. Moreover, the instant communication software is flexible to apply, and can be used for sharing and ranking the record information.

A receiving module 160, used for receiving the second user terminal record information of a similar intelligent device of a second user associated with the instant communication software account number and returned by the server. The manner of associating the instant communication software account number with the second user may be set according to the demands of the users, for example, the friends in the instant communication, the users within a certain range determinate by the instant communication software, and friends in a group having the same interests and hobbies, and the like.

In one embodiment, a plurality of second users are included. The receiving module 160 may receive a predetermined number of top-ranked second user terminal record information among the second user terminal record information of the similar intelligent device of the second user associated with the instant communication software account number and returned by the server. In the embodiment, the second user terminal record information is firstly screened, and only the second user terminal record information ranked in the top five, top ten or the like of second user terminal record information are received, so that the quantity of information received may be reduced, and the network bandwidth is saved.

A sorting module 180, used for sorting and displaying the first user terminal record information and the second user terminal record information. Preferably, the embodiment may further be used for receiving the ranking information of the first user terminal record information returned by the server. The first user may know the rank in the relationship chain thereof if not in the top rank of the predetermined quantity. More preferably, the embodiment may comprise the step of receiving a predetermined number of second user terminal record information among a plurality of second user terminal record information, which are close to the rank of the first user terminal record information and returned by the server, thus being convenient for the first user to know the second user terminal record information ranked close to the first user, know the difference surrounding thereof, and having an encourage effect on the first user.

Referring to FIG. 8, one embodiment therein further comprises a binding module 110, used for binding the intelligent device with the instant communication software account number. The manners of binding the intelligent device with the instant communication software account number comprise one or combinations of acquisition through Bluetooth, acquisition through a wireless near field communication technology NFC, acquisition through two-dimensional code scanning, and acquisition through word information or digital information inputting. The Bluetooth manner and the NFC manner may be the same with the manner of acquiring the first user terminal record information of the intelligent device, which are more convenient and faster. The manner of acquisition through two-dimensional code scanning and the manner of acquisition through word information or digital information inputting can save more cost as a wireless communication protocol used for binding is not needed.

A person having ordinary skill in the art may understand that all or part of the flow for implementing the methods according to the foregoing embodiments are programs that may be executed by relational hardware instructed by a computer program, and may be stored in a computer readable storage medium. The program when executed may complete the flow of the methods according to the foregoing embodiments. The storage medium may be a magnetic disk, an optical disk, a read-only memory (ROM) or a random access memory (RAM), and the like.

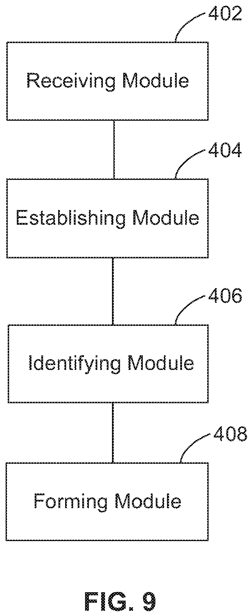

FIG. 9 is a block diagram illustrative of modules in a server in accordance with some embodiments. As shown in FIG. 9, the server may comprise a receiving module 402, an establishing module 404, an identifying module 406, and a forming module 408.

The receiving module 402 is configured to receive a first device identifier associated with a first health monitoring device from a first user terminal. In some embodiments, the first user terminal is configured to communicate with the first health monitoring device to receive health monitoring information of a respective monitored subject of the first health monitoring device.

The establishing module 404 is configured to establish a corresponding relationship between the first device identifier and a first user ID of the social networking platform. In some embodiments, the first user ID corresponds to a first user associated with the first user terminal.

The identifying module 406 is configured to identify at least one second user ID from a plurality of social network contacts of the first user ID based on the first device identifier. In some embodiments, each of the at least one second user ID is associated with a respective second device identifier for a respective second health monitoring device that provides health monitoring information of a respective monitored subject of the respective second health monitoring device.

The forming module 408 is configured to form a respective social network group including the first user ID and the at least one second user ID for sharing the health monitoring information received from the first health monitoring device and the respective second health monitoring device of the at least one second user ID.

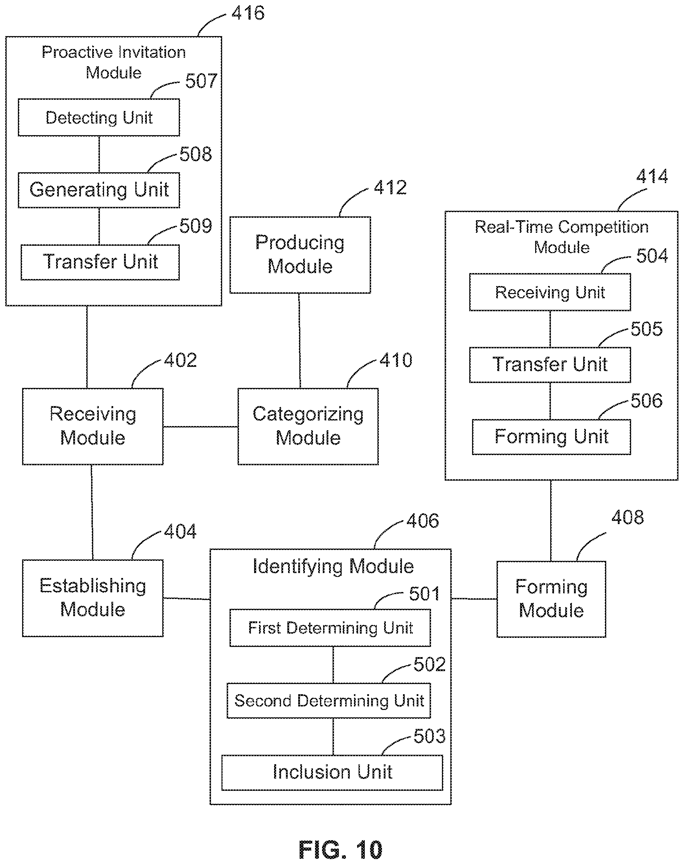

FIG. 10 is a block diagram illustrative of modules in a server in accordance with some embodiments of the current application. As shown in FIG. 10, the server may further comprise a categorizing module 410, a producing module 412, and a real-time competition module 414, in addition to the receiving module 402, the establishing module 404, the identifying module 406, and the forming module 408.

In some embodiments, the identifying module 406 comprises a first determining unit 501, a second determining unit 502, and an inclusion unit 503.

The first determining unit 501 is configured to determine a respective device type for the first health monitoring device based on the first device identifier. For each of the plurality of social network contacts of the first user ID: the second determining unit 502 is configured to determine whether the social network contact is associated with a respective device identifier for a device of the respective device type; and the inclusion unit 503 is configured to include the social network contact as one of the at least one second user ID in accordance with a determination that the social network contact and the first user ID are both associated with the respective device identifiers of the same device type.

In some embodiments, the receiving module 402 is further configured to receive the first device identifier, the first user ID, and first health monitoring information from the first user terminal, wherein the first health monitoring information is collected from the first health monitoring device by the first user terminal.

The categorizing module 410 is configured to categorize the first health monitoring information based on at least the first device identifier or parameters extracted from the first health monitoring information.

The producing module 412 configured to produce a sharing list of health monitoring information including the first health monitoring information received from the first user terminal, wherein the sharing list is accessible by the respective social network group.

The real-time competition module 414 comprises a receiving unit 504, a transfer unit 505, and a forming unit 506. The real-time competition module 414 is configured to manage the formation of competition groups for real-time competition and the sharing of health monitoring information related to real-time competition.

In some embodiments, the receiving unit 504 is configured to receive an invitation request from the first user terminal to one or more connected users in the respective social network group for a real-time competition related to an activity.

In some embodiments, the transfer unit 505 is configured to send a respective invitation to each of the one or more connected users in accordance with the invitation request.

In some embodiments, the forming unit 506 is configured to form a competition group including the first user ID and one or more connected users that have responded to their respective invitations.

The proactive invitation module 416 comprises a detecting unit 507, a generating unit 508, and a transfer unit 509. The proactive invitation module 416 is configured to proactively detect real-time health monitoring information and send invitations to user terminals to participate in a real-time competition.

In some embodiments, the detecting unit 507 is configured to detect transmission of real-time health monitoring information from respective user terminals of two or more user IDs in the respective social network group; and

In some embodiments, the generating unit 508 is configured to, proactively in response to detecting the transmission, without request from the respective user terminals of the two or more user IDs in the respective social network group, generate an invitation for the two or more user IDs to participate in a real-time competition based on an activity monitored by the real-time health monitoring information.

In some embodiments, the transfer unit 509 is configured to send the invitation to each of the two or more user IDs.

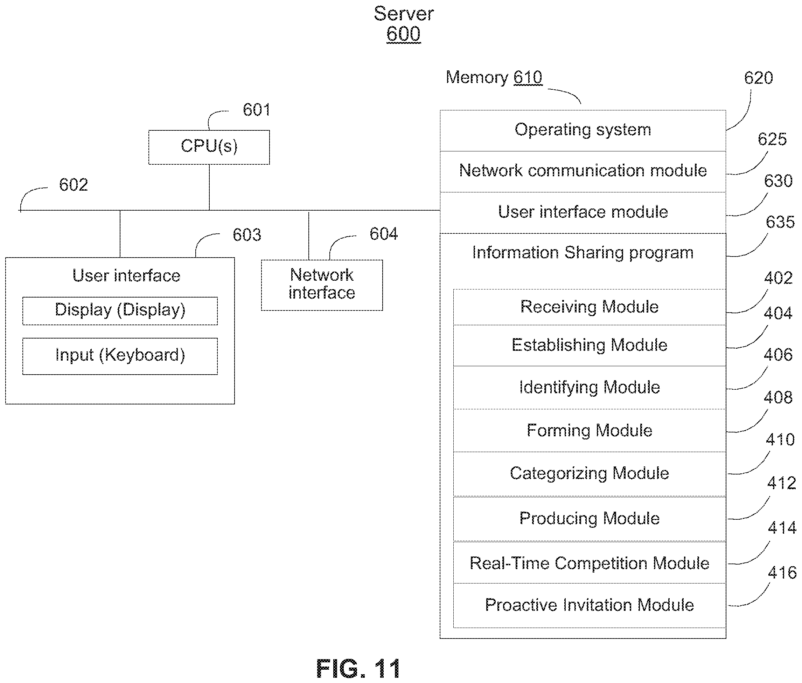

FIG. 11 is a schematic diagram of a server in accordance with some embodiments of the current application. The exemplary server 600 typically includes one or more processing units (CPU's) 601, one or more network or other communications interfaces 604, memory 610, and one or more communication buses 602 for interconnecting these components. The communication buses 602 may include circuitry (sometimes called a chipset) that interconnects and controls communications between system components. The first user terminal 600 may include a user interface 603, for instance, a display and a keyboard. When the first user terminal 600 is a smart phone or tablet, the user interface 603 may be a touch screen, which is both a display and an input device. Memory 610 may include high speed random access memory and may also include non-volatile memory, such as one or more magnetic disk storage devices. Memory 610 may include mass storage that is remotely located from the CPU's 601. In some embodiments, memory 610 stores the following programs, modules and data structures, or a subset or superset thereof:
- an operating system 620 that includes procedures for handling various basic system services and for performing hardware dependent tasks;
- a network communication module 625 that is used for connecting the server 600 to user terminals and/or other computers via one or more communication networks (wired or wireless), such as the Internet, other wide area networks, local area networks, metropolitan area networks, and so on;
- a user interface module 630 configured to receive user inputs through the user interface 603;
- and a number of information sharing program modules 635 including the following:
   - a receiving module 402 configured to receive a first device identifier associated with a first health monitoring device from a first user terminal;
   - an establishing module 404 configured to establish a corresponding relationship between the first device identifier and a first user ID of the social networking platform;
   - an identifying module 406 configured to identify at least one second user ID from a plurality of social network contacts of the first user ID based on the first device identifier;
   - a forming module 408 configured to form a respective social network group including the first user ID and the at least one second user ID for sharing the health monitoring information received from the first health monitoring device and the respective second health monitoring device of the at least one second user ID;
   - a categorizing module 410 configured to categorize the first health monitoring information based on at least the first device identifier or parameters extracted from the first health monitoring information;
   - a producing module 412 configured to produce a sharing list of health monitoring information including the first health monitoring information received from the first user terminal, wherein the sharing list is accessible by the respective social network group;
   - a real-time competition module 414 configured to manage the formation of competition groups for real-time competition and the sharing of health monitoring information related to real-time competition; and
   - a proactive invitation module 416 configured to proactively detect real-time health monitoring information and send invitations to user terminals to participate in a real-time competition.

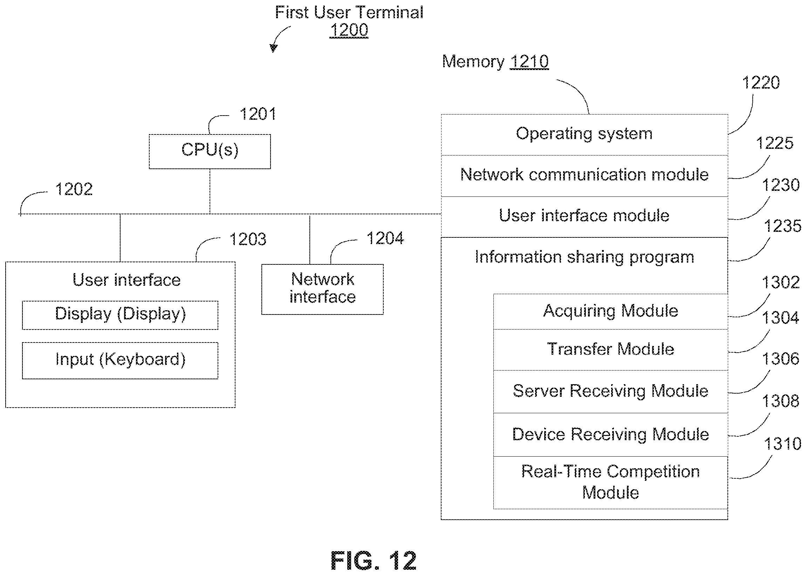

FIG. 12 is a schematic diagram of a first user terminal in accordance with some embodiments of the current application. The exemplary first user terminal 1200 typically includes one or more processing units (CPU's) 1201, one or more network or other communications interfaces 1204, memory 1210, and one or more communication buses 1202 for interconnecting these components. The communication buses 1202 may include circuitry (sometimes called a chipset) that interconnects and controls communications between system components. The first user terminal 1200 may include a user interface 1203, for instance, a display and a keyboard. When the first user terminal 1200 is a smart phone or tablet, the user interface 1203 may be a touch screen, which is both a display and an input device. Memory 1210 may include high speed random access memory and may also include non-volatile memory, such as one or more magnetic disk storage devices. Memory 1210 may include mass storage that is remotely located from the CPU's 1201. In some embodiments, memory 1210 stores the following programs, modules and data structures, or a subset or superset thereof:
- an operating system 1220 that includes procedures for handling various basic system services and for performing hardware dependent tasks;
- a network communication module 1225 that is used for connecting the first user terminal 1200 to the server and other user terminals, and/or other computers via one or more communication networks (wired or wireless), such as the Internet, other wide area networks, local area networks, metropolitan area networks, and so on;

- a user interface module 1230 configured to receive user inputs through the user interface 1203;
- and a number of information sharing program modules 1235 including the following:
   - an acquiring module 1302 configured to acquire a first device identifier associated with a first health monitoring device through a communication program installed on the first user terminal, wherein the communication program is configured to communicate with a server of a social network platform using a first user ID, and the communication program is further configured to receive health monitoring information of a respective monitored subject of the first health monitoring device;
   - a transfer module 1304 configured to send a request to the server to bind the first device identifier with the first user ID;
   - a server receiving module 1306 configured to receive a notification from the server regarding a respective social network group in response to sending the request, wherein the notification is for sharing the health monitoring information received from the first health monitoring device with at least one second user ID identified from a plurality of social network contacts of the first user ID;
   - a device receiving module 1308 configured to receive first health monitoring information from the first health monitoring device based on monitored activities of a first user associated with the first user terminal, wherein the respective social network group includes one or more second user IDs identified from the plurality of social network contacts of the first user ID that are each associated with a respective second device identifier for a respective second health monitoring device that provides health monitoring information of a respective monitored subject of the respective second health monitoring device; and
   - a real-time competition module 1310 configured to manage the first user terminal's participation in a real-time competition and data sharing.

## Claims

1. A method of information sharing comprising:
at a server of a social network platform having one or more processors and memory storing programs executed by the one or more processors,
receiving a first device identifier associated with a first health monitoring device from a first user terminal, the first user terminal configured to communicate with the first health monitoring device to receive health monitoring information of a respective monitored subject of the first health monitoring device (S410);
establishing a corresponding relationship between the first device identifier and a first user ID of the social networking platform, wherein the first user ID corresponds to a first user associated with the first user terminal (S420);
identifying at least one second user ID from a plurality of social network contacts of the first user ID based on the first device identifier, wherein each of the at least one second user ID is associated with a respective second device identifier for a respective second health monitoring device that provides health monitoring information of a respective monitored subject of the respective second health monitoring device (S430); and
forming a respective social network group including the first user ID and the at least one second user ID for sharing the health monitoring information received from the first health monitoring device and the respective second health monitoring device of the at least one second user ID (S440); **characterized in that** identifying the at least one second user ID based on the first device identifier further comprises:
determining a respective device type for the first health monitoring device based on the first device identifier;
for each of the plurality of social network contacts of the first user ID:
determining whether the social network contact is associated with a respective device identifier for a device of the respective device type; and
in accordance with a determination that the social network contact and the first user ID are both associated with the respective device identifiers of the same device type, including the social network contact as one of the at least one second user ID.

2. The method of claim 1, wherein the first device identifier is associated with at least two device types or at least two exercise data types, and wherein forming the respective social network group including the first user ID and the at least one second user ID further comprises:
forming a respective social network group for each of the at least two device types or each of the at least two exercise data types, wherein the respective social network group includes the first user ID and one or more of the at least one second user ID that correspond to the same device type or exercise data type.

3. The method of claim 1, further comprising:
receiving the first device identifier, the first user ID, and first health monitoring information from the first user terminal, wherein the first health monitoring information is collected from the first health monitoring device by the first user terminal (S450);
categorizing the first health monitoring information based on at least the first device identifier or parameters extracted from the first health monitoring information (S460); and
sharing the first health monitoring information in the respective social network group (S470).

4. The method of claim 1, wherein the plurality of social network contacts of the first user ID consists of all user IDs that are contacts of at least a predetermined degree to the first user ID.

5. The method of claim 1, further comprising:
receiving an invitation request from the first user terminal to one or more connected users in the respective social network group for a real-time competition related to an activity (S510); and
sending a respective invitation to each of the one or more connected users in accordance with the invitation request (S520), and
forming a competition group including the first user ID and one or more connected users that have responded to their respective invitations (S530).

6. The method of claim 5, further comprising:
during the real-time competition:
receiving respective real-time health monitoring information from each user participating in the competition group (S540), and
sharing competition information extracted from the real-time health monitoring information received from each user participating in the competition group with other users in the competition group (S550).

7. The method of claim 6, wherein sharing the competition information extracted from the real-time health monitoring information received from each user participating in the competition group with other users in the competition group further comprises:
providing real-time comparison of the competition information extracted from the real-time health monitoring information received from each user participating in the competition group to each user participating in the competition group.

8. The method of claim 6, wherein sharing the competition information extracted from the real-time health monitoring information received from each user participating in the competition group with other users in the competition group further comprises:
providing a final comparison of the competition information extracted from the real-time health monitoring information received from each user participating in the competition group to each user participating in the competition group.

9. The method of claim 1, further comprising:
detecting transmission of real-time health monitoring information from respective user terminals of two or more user IDs in the respective social network group;
in response to detecting the transmission, proactively, without request from the respective user terminals of the two or more user IDs in the respective social network group, generating an invitation for the two or more user IDs to participate in a real-time competition based on an activity monitored by the real-time health monitoring information; and
sending the invitation to each of the two or more user IDs.

10. A server comprising:
one of more processors;
memory; and
one or more programs modules stored in the memory and configured for execution by the one or more processors, the one or more program modules including:
a receiving module (160) configured to receive a first device identifier associated with a first health monitoring device from a first user terminal, the first user terminal configured to communicate with the first health monitoring device to receive health monitoring information of a respective monitored subject of the first health monitoring device;
an establishing module (404) configured to establish a corresponding relationship between the first device identifier and a first user ID of the social networking platform, wherein the first user ID corresponds to a first user associated with the first user terminal;
an identifying module (406) configured to identify at least one second user ID from a plurality of social network contacts of the first user ID based on the first device identifier, wherein each of the at least one second user ID is associated with a respective second device identifier for a respective second health monitoring device that provides health monitoring information of a respective monitored subject of the respective second health monitoring device; and
a forming module (408) configured to form a respective social network group including the first user ID and the at least one second user ID for sharing the health monitoring information received from the first health monitoring device and the respective second health monitoring device of the at least one second user ID; **characterized in that** the identifying module (406) comprises a first determining unit, a second determining unit, and an inclusion unit,
the first determining unit (501) is configured to determine a respective device type for the first health monitoring device based on the first device identifier;
for each of the plurality of social network contacts of the first user ID:
the second determining unit (502) is configured to determine whether the social network contact is associated with a respective device identifier for a device of the respective device type; and
the inclusion unit (503) is configured to include the social network contact as one of the at least one second user ID in accordance with a determination that the social network contact and the first user ID are both associated with the respective device identifiers of the same device type.

11. The server of claim 10, wherein:
the receiving module (160) is further configured to receive the first device identifier, the first user ID, and first health monitoring information from the first user terminal, wherein the first health monitoring information is collected from the first health monitoring device by the first user terminal;
the server further comprises a categorizing module (410) configured to categorize the first health monitoring information based on at least the first device identifier or parameters extracted from the first health monitoring information; and
the server further comprises a producing module (412) configured to produce a sharing list of health monitoring information including the first health monitoring information received from the first user terminal, wherein the sharing list is accessible by the respective social network group.

12. The server of claim 10, further comprising a real-time competition module (414), wherein:
the real-time competition module (414) comprises a receiving unit, a transfer unit, and a forming unit,
the receiving unit (402) is configured to receive an invitation request from the first user terminal to one or more connected users in the respective social network group for a real-time competition related to an activity; and
the transfer unit (505) is configured to send a respective invitation to each of the one or more connected users in accordance with the invitation request, and
the forming unit (506) is configured to form a competition group including the first user ID and one or more connected users that have responded to their respective invitations.

13. The server of claim 10, further comprising a proactive invitation module (416), wherein:
the proactive invitation module (416) comprises a detecting unit, a generating unit, and a transfer unit,
the detecting unit (507) is configured to detect transmission of real-time health monitoring information from respective user terminals of two or more user IDs in the respective social network group; and
the generating unit (508) is configured to, proactively in response to detecting the transmission, without request from the respective user terminals of the two or more user IDs in the respective social network group, generate an invitation for the two or more user IDs to participate in a real-time competition based on an activity monitored by the real-time health monitoring information; and;
the transfer unit (509) is configured to send the invitation to each of the two or more user IDs.

## Patentansprüche

1. Verfahren zum Teilen von Informationen, das umfasst:
Auf einem Server einer sozialen Netzwerkplattform mit einem oder mehreren Prozessoren und einem Speicher, der Programme speichert, die von dem einen oder den mehreren Prozessoren ausgeführt werden,
Empfangen einer ersten Vorrichtungskennung, die einer ersten Gesundheitsüberwachungsvorrichtung zugeordnet ist, von einem ersten Benutzerendgerät, wobei das erste Benutzerendgerät so eingerichtet ist, dass es mit der ersten Gesundheitsüberwachungsvorrichtung kommuniziert, um Gesundheitsüberwachungsinformationen eines jeweiligen überwachten Probanden der ersten Gesundheitsüberwachungsvorrichtung (S410) zu empfangen;
Herstellen einer entsprechenden Beziehung zwischen der ersten Vorrichtungskennung und einer ersten Benutzer-ID der Plattform für soziale Netzwerke, wobei die erste Benutzer-ID einem ersten Benutzer entspricht, der dem ersten Benutzerendgerät zugeordnet ist (S420);
Identifizieren von wenigstens einer zweiten Benutzer-ID aus einer Vielzahl von Kontakten des sozialen Netzwerks der ersten Benutzer-ID basierend auf der ersten Vorrichtungskennung, wobei jede der wenigstens einen zweiten Benutzer-ID einer jeweiligen zweiten Vorrichtungskennung für eine jeweilige zweite Gesundheitsüberwachungsvorrichtung zugeordnet ist, das Gesundheitsüberwachungsinformationen eines jeweiligen überwachten Probanden der jeweiligen zweiten Gesundheitsüberwachungsvorrichtung (S430) bereitstellt; und
Bilden einer jeweiligen Gruppe des sozialen Netzwerks, die die erste Benutzer-ID und die wenigstens eine zweite Benutzer-ID einschließt, um die von der ersten Gesundheitsüberwachungsvorrichtung und der jeweiligen zweiten Gesundheitsüberwachungsvorrichtung der wenigstens einen zweiten Benutzer-ID empfangenen Gesundheitsüberwachungsinformationen gemeinsam zu nutzen (S440); **dadurch gekennzeichnet, dass** das Identifizieren der wenigstens einen zweiten Benutzer-ID basierend auf der ersten Vorrichtungskennung ferner umfasst:
Bestimmen eines entsprechenden Vorrichtungstyps für die erste Gesundheitsüberwachungsvorrichtung basierend auf der ersten Vorrichtungskennung;
für jeden der Vielzahl von Kontakten des sozialen Netzwerks der ersten Benutzer-ID:
Bestimmen, ob der Kontakt des sozialen Netzwerks einer entsprechenden Vorrichtungskennung für eine Vorrichtung des entsprechenden Vorrichtungstyps zugeordnet ist; und
in Übereinstimmung mit dem Bestimmen, dass der Kontakt des sozialen Netzwerks und die erste Benutzer-ID beide mit den jeweiligen Vorrichtungskennungen desselben Vorrichtungstyps verbunden sind, einschließlich des Kontakts des sozialen Netzwerks als eine der wenigstens einen zweiten Benutzer-ID.

2. Verfahren nach Anspruch 1, wobei die erste Vorrichtungskennung wenigstens zwei Vorrichtungstypen oder wenigstens zwei Übungsdatentypen zugeordnet ist, und wobei das Bilden der jeweiligen Gruppe des sozialen Netzwerks, die die erste Benutzer-ID und die wenigstens eine zweite Benutzer-ID einschließt, ferner umfasst:
Bilden einer jeweiligen Gruppe des sozialen Netzwerks für jeden der wenigstens zwei Vorrichtungstypen oder jeden der wenigstens zwei Übungsdatentypen, wobei die jeweilige Gruppe des sozialen Netzwerks die erste Benutzer-ID und eine oder mehrere der wenigstens einen zweiten Benutzer-ID einschließt, die demselben Vorrichtungstyp oder Übungsdatentyp entsprechen.

3. Verfahren nach Anspruch 1, das ferner umfasst:
Empfangen der ersten Vorrichtungskennung, der ersten Benutzer-ID und der ersten Gesundheitsüberwachungsinformationen von dem ersten Benutzerendgerät, wobei die ersten Gesundheitsüberwachungsinformationen von der ersten Gesundheitsüberwachungsvorrichtung durch das erste Benutzerendgerät gesammelt werden (S450);
Kategorisieren der ersten Gesundheitsüberwachungsinformationen basierend auf wenigstens der ersten Vorrichtungskennung oder von Parametern, die aus den ersten Gesundheitsüberwachungsinformationen (S460) extrahiert wurden; und
Teilen der ersten Gesundheitsüberwachungsinformationen in der jeweiligen Gruppe des sozialen Netzwerks (S470).

4. Verfahren nach Anspruch 1, wobei die Vielzahl von Kontakten des sozialen Netzwerks der ersten Benutzer-ID aus allen Benutzer-IDs besteht, die Kontakte mit wenigstens einem vorbestimmten Grad zu der ersten Benutzer-ID sind.

5. Verfahren nach Anspruch 1, das ferner umfasst:
Empfangen einer Einladungsanfrage von dem ersten Benutzerendgerät an einen oder mehrere verbundene Benutzer in der jeweiligen Gruppe des sozialen Netzwerks für einen Echtzeitwettbewerb in Bezug auf eine Aktivität (S510); und
Senden einer jeweiligen Einladung an jeden der einen oder mehreren verbundenen Benutzer in Übereinstimmung mit der Einladungsanfrage (S520), und
Bilden einer Wettbewerbsgruppe, die die erste Benutzer-ID und einen oder mehrere verbundene Benutzer einschließt, die auf ihre jeweiligen Einladungen (S530) geantwortet haben.

6. Verfahren nach Anspruch 5, das ferner umfasst:
während des Echtzeitwettbewerbs:
Empfangen jeweiliger Echtzeit-Gesundheitsüberwachungsinformationen von jedem an der Wettbewerbsgruppe teilnehmenden Benutzer (S540), und
Teilen von Wettbewerbsinformationen, die aus den Echtzeit-Gesundheitsüberwachungsinformationen extrahiert wurden, die von jedem an der Wettbewerbsgruppe teilnehmenden Benutzer empfangen wurden, mit anderen Benutzern in der Wettbewerbsgruppe (S550).

7. Verfahren nach Anspruch 6, wobei das Teilen der Wettbewerbsinformationen, die aus den Echtzeit-Gesundheitsüberwachungsinformationen extrahiert wurden, die von jedem an der Wettbewerbsgruppe teilnehmenden Benutzer empfangen wurden, mit anderen Benutzern in der Wettbewerbsgruppe ferner umfasst:
Bereitstellen eines Echtzeitvergleichs der Wettbewerbsinformationen, die aus den Echtzeit-Gesundheitsüberwachungsinformationen extrahiert wurden, die von jedem an der Wettbewerbsgruppe teilnehmenden Benutzer empfangen wurden, mit jedem an der Wettbewerbsgruppe teilnehmenden Benutzer.

8. Verfahren nach Anspruch 6, wobei das Teilen der Wettbewerbsinformationen, die aus den Echtzeit-Gesundheitsüberwachungsinformationen extrahiert wurden, die von jedem an der Wettbewerbsgruppe teilnehmenden Benutzer empfangen wurden, mit anderen Benutzern in der Wettbewerbsgruppe ferner umfasst:
Bereitstellen eines endgültigen Vergleichs der Wettbewerbsinformationen, die aus den Echtzeit-Gesundheitsüberwachungsinformationen extrahiert wurden, die von jedem an der Wettbewerbsgruppe teilnehmenden Benutzer empfangen wurden, mit jedem an der Wettbewerbsgruppe teilnehmenden Benutzer.

9. Verfahren nach Anspruch 1, ferner umfassend:
Erfassen der Übertragung von Echtzeit-Gesundheitsüberwachungsinformationen von den jeweiligen Benutzerendgeräten von zwei oder mehr Benutzer-IDs in der jeweiligen Gruppe des sozialen Netzwerks;
als Reaktion auf das Erfassen der Übertragung proaktiv, ohne Anfrage von den jeweiligen Benutzerendgeräts der zwei oder mehr Benutzer-IDs in der jeweiligen Gruppe des sozialen Netzwerks, Erzeugen einer Einladung für die zwei oder mehr Benutzer-IDs zur Teilnahme an einem Echtzeitwettbewerb basierend auf einer durch die Echtzeit-Gesundheitsüberwachungsinformationen überwachten Aktivität; und
Senden der Einladung an jede der zwei oder mehr Benutzer-IDs.

10. Server, umfassend:
einen oder mehrere Prozessoren;
Speicher; und
ein oder mehrere Programmmodule, die in dem Speicher gespeichert und zur Ausführung durch den einen oder die mehreren Prozessoren eingerichtet sind, wobei das eine oder die mehreren Programmmodule einschließen:
ein Empfangsmodul (160), das so eingerichtet ist, dass es eine erste Vorrichtungskennung, die mit einer ersten Gesundheitsüberwachungsvorrichtung verbunden ist, von einem ersten Benutzerendgerät empfängt, wobei das erste Benutzerendgerät so eingerichtet ist, dass es mit der ersten Gesundheitsüberwachungsvorrichtung kommuniziert, um Gesundheitsüberwachungsinformationen eines jeweiligen überwachten Probanden der ersten Gesundheitsüberwachungsvorrichtung zu empfangen;
ein Einrichtungsmodul (404), das so eingerichtet ist, dass es eine entsprechende Beziehung zwischen der ersten Vorrichtungskennung und einer ersten Benutzer-ID der Plattform für soziale Netzwerke herstellt, wobei die erste Benutzer-ID einem ersten Benutzer entspricht, der mit dem ersten Benutzerendgerät verbunden ist;
ein Identifizierungsmodul (406), das so eingerichtet ist, dass es wenigstens eine zweite Benutzer-ID aus einer Vielzahl von Kontakten des sozialen Netzwerks der ersten Benutzer-ID basierend auf der ersten Vorrichtungskennung identifiziert, wobei jede der wenigstens einen zweiten Benutzer-ID mit einer jeweiligen zweiten Vorrichtungskennung für eine jeweilige zweite Gesundheitsüberwachungsvorrichtung verbunden ist, die Gesundheitsüberwachungsinformationen eines jeweiligen überwachten Probanden der jeweiligen zweiten Gesundheitsüberwachungsvorrichtung bereitstellt; und
ein Bildungsmodul (408), das so eingerichtet ist, dass es eine jeweilige Gruppe des sozialen Netzwerks bildet, die die erste Benutzer-ID und die wenigstens eine zweite Benutzer-ID einschließt, um die von der ersten Gesundheitsüberwachungsvorrichtung und der jeweiligen zweiten Gesundheitsüberwachungsvorrichtung der wenigstens einen zweiten Benutzer-ID empfangenen Gesundheitsüberwachungsinformationen zu teilen; **dadurch gekennzeichnet, dass** das Identifizierungsmodul (406) eine erste Bestimmungseinheit, eine zweite Bestimmungseinheit und eine Einschließungseinheit umfasst,
die erste Bestimmungseinheit (501) so eingerichtet ist, dass sie einen jeweiligen Vorrichtungstyp für die erste Gesundheitsüberwachungsvorrichtung basierend auf der ersten Vorrichtungskennung bestimmt;
für jeden der Vielzahl von Kontakten des sozialen Netzwerks der ersten Benutzer-ID:
wobei die zweite Bestimmungseinheit (502) so eingerichtet ist, dass sie bestimmt, ob der Kontakt des sozialen Netzwerks mit einer jeweiligen Vorrichtungskennung für eine Vorrichtung des jeweiligen Vorrichtungstyps verbunden ist; und
die Einschließungseinheit (503) so eingerichtet ist, dass sie den Kontakt des sozialen Netzwerks als eine der wenigstens einen zweiten Benutzer-ID in Übereinstimmung mit einer Bestimmung einschließt, dass der Kontakt des sozialen Netzwerks und die erste Benutzer-ID beide mit den jeweiligen Vorrichtungskennungen desselben Vorrichtungstyps verbunden sind.

11. Server nach Anspruch 10, wobei:
das Empfangsmodul (160) ferner so eingerichtet ist, dass es die erste Vorrichtungskennung, die erste Benutzer-ID und erste Gesundheitsüberwachungsinformationen von dem ersten Benutzerendgerät empfängt, wobei die ersten Gesundheitsüberwachungsinformationen von der ersten Gesundheitsüberwachungsvorrichtung durch das erste Benutzerendgerät gesammelt werden;
der Server ferner ein Kategorisierungsmodul (410) umfasst, das so eingerichtet ist, dass es die ersten Gesundheitsüberwachungsinformationen basierend auf wenigstens der ersten Vorrichtungskennung oder von Parametern, die aus den ersten Gesundheitsüberwachungsinformationen extrahiert wurden, kategorisiert; und
der Server ferner ein Erzeugungsmodul (412) umfasst, das eingerichtet ist, um eine Freigabeliste von Gesundheitsüberwachungsinformationen zu erzeugen, die die von dem ersten Benutzerendgerät empfangenen ersten Gesundheitsüberwachungsinformationen einschließt, wobei die Freigabeliste für die jeweilige Gruppe des sozialen Netzwerks zugänglich ist.

12. Server nach Anspruch 10, der ferner ein Echtzeitwettbewerbsmodul (414) umfasst, wobei:
das Echtzeitwettbewerbsmodul (414) eine Empfangseinheit, eine Übertragungseinheit und eine Bildungseinheit umfasst,
die Empfangseinheit (402) so eingerichtet ist, dass sie eine Einladungsanfrage von dem ersten Benutzerendgerät an einen oder mehrere verbundene Benutzer in der jeweiligen Gruppe des sozialen Netzwerks für einen Echtzeitwettbewerb in Bezug auf eine Aktivität empfängt; und
die Übertragungseinheit (505) so eingerichtet ist, dass sie entsprechend der Einladungsanfrage eine jeweilige Einladung an jeden der einen oder mehreren verbundenen Benutzer sendet, und
die Bildungseinheit (506) so eingerichtet ist, dass sie eine Wettbewerbsgruppe bildet, die die erste Benutzer-ID und einen oder mehrere verbundene Benutzer einschließt, die auf ihre jeweiligen Einladungen geantwortet haben.

13. Server nach Anspruch 10, der ferner ein proaktives Einladungsmodul (416) umfasst, wobei:
das proaktive Einladungsmodul (416) eine Erfassungseinheit, eine Erzeugungseinheit und eine Übertragungseinheit umfasst,
die Erfassungseinheit (507) so eingerichtet ist, dass sie die Übertragung von Echtzeit-Gesundheitsüberwachungsinformationen von jeweiligen Benutzerendgeräten von zwei oder mehr Benutzer-IDs in der jeweiligen Gruppe des sozialen Netzwerks erfasst; und
die Erzeugungseinheit (508) so eingerichtet ist, dass sie proaktiv als Reaktion auf das Erfassen der Übertragung ohne Anfrage von den jeweiligen Benutzerendgeräten der zwei oder mehr Benutzer-IDs in der jeweiligen Gruppe des sozialen Netzwerks eine Einladung für die zwei oder mehr Benutzer-IDs zur Teilnahme an einem Echtzeitwettbewerb basierend auf einer durch die Echtzeit-Gesundheitsüberwachungsinformationen überwachten Aktivität erzeugt; und;
die Übertragungseinheit (509) so eingerichtet ist, dass sie die Einladung an jede der zwei oder mehr Benutzer-IDs sendet.

## Revendications

1. Procédé de partage d'informations comprenant :
au niveau d'un serveur d'une plate-forme de réseau social ayant un ou plusieurs processeurs et des programmes de stockage en mémoire exécutés par les un ou plusieurs processeurs,
de recevoir un premier identificateur de dispositif associé à un premier dispositif de surveillance de santé depuis un premier terminal utilisateur, le premier terminal utilisateur configuré pour communiquer avec le premier dispositif de surveillance de santé pour recevoir des informations de surveillance de santé d'un sujet surveillé respectif du premier dispositif de surveillance de santé (S410) ;
d'établir une relation correspondante entre le premier identificateur de dispositif et un premier ID utilisateur de la plate-forme de réseau social, dans lequel le premier ID utilisateur correspond à un premier utilisateur associé au premier terminal d'utilisateur (S420) ;
d'identifier au moins un second ID utilisateur dans une pluralité de contacts de réseaux sociaux du premier ID utilisateur en se basant sur le premier identificateur de dispositif, dans lequel chacun des au moins un second ID utilisateur est associé à un second identificateur de dispositif respectif pour un second dispositif de surveillance de santé respectif qui fournit des informations de surveillance de santé d'un sujet surveillé du second dispositif de surveillance de santé respectif (S430) ; et
de former un groupe de réseau social respectif incluant le premier ID utilisateur et l'au moins un second ID utilisateur pour partager les informations de surveillance de santé reçues depuis le premier dispositif de surveillance de santé et le second dispositif de surveillance de santé respectif de l'au moins un second ID utilisateur (S440) ; **caractérisé en ce qu'**identifier l'au moins un second ID utilisateur en se basant sur le premier identificateur de dispositif comprend en outre :
de déterminer un type de dispositif respectif pour le premier dispositif de surveillance de santé en se basant sur le premier identificateur de dispositif ;
pour chacun de la pluralité de contacts de réseaux sociaux du premier ID utilisateur :
de déterminer si le contact de réseau social est associé à un identificateur de dispositif respectif pour un dispositif du type de dispositif respectif ; et
selon une détermination que le contact de réseau social et le premier ID utilisateur sont tous deux associés aux identificateurs de dispositif respectifs du même type de dispositif, d'inclure le contact de réseau social comme l'un de l'au moins un second ID utilisateur.

2. Procédé selon la revendication 1, dans lequel le premier identificateur de dispositif est associé à au moins deux types de dispositif ou au moins deux types de données d'entraînement, et dans lequel former le groupe de réseau social respectif incluant le premier ID utilisateur et l'au moins un second ID utilisateur comprend en outre :
de former un groupe de réseau social respectif pour chacun des au moins deux types de dispositif ou chacun des au moins deux types de données d'entraînement, dans lequel le groupe de réseau social respectif inclut le premier ID utilisateur et un ou plusieurs des au moins un second ID utilisateur qui correspondent au même type de dispositif ou type de données d'entraînement.

3. Procédé selon la revendication 1, comprenant en outre :
de recevoir le premier identificateur de dispositif, le premier ID utilisateur, et les premières informations de surveillance de santé depuis le premier terminal utilisateur, dans lequel les premières informations de surveillance de santé sont collectées depuis le premier dispositif de surveillance de santé par le premier terminal utilisateur (S450) ;
de catégoriser les premières informations de surveillance de santé en se basant sur au moins le premier identificateur de dispositif ou des paramètres extraits des premières informations de surveillance de santé (S460) ; et
de partager les premières informations de surveillance de santé dans le groupe de réseau social respectif (S470).

4. Procédé selon la revendication 1, dans lequel la pluralité de contacts de réseaux sociaux du premier ID utilisateur consiste en toutes les ID utilisateur qui sont des contacts d'au moins un degré prédéterminé du premier ID utilisateur.

5. Procédé selon la revendication 1, comprenant en outre
de recevoir une requête d'invitation depuis le premier terminal utilisateur vers un ou plusieurs utilisateurs connectés dans le groupe de réseau social respectif pour une compétition en temps réel liée à une activité (S510) ; et
d'envoyer une invitation respective à chacun des un ou plusieurs utilisateurs connectés selon la requête d'invitation (S520) ; et
de former un groupe de compétition incluant le premier ID utilisateur et un ou plusieurs utilisateurs connectés qui ont répondu à leurs invitations respectives (S530).

6. Procédé selon la revendication 5, comprenant en outre pendant la compétition en temps réel :
de recevoir des informations de surveillance de santé en temps réel de chaque utilisateur participant dans le groupe de compétition (S540), et
de partager des informations de compétition extraites des informations de surveillance de santé en temps réel reçues de chaque utilisateur participant dans le groupe de compétition avec d'autres utilisateurs dans le groupe de compétition (S550).

7. Procédé selon la revendication 6, dans lequel partager les informations de compétition extraites des informations de surveillance de santé en temps réel reçues de chaque utilisateur participant dans le groupe de compétition avec d'autres utilisateurs dans le groupe de compétition comprend en outre :
de fournir une comparaison en temps réel des informations de compétition extraite des informations de surveillance de santé reçues de chaque utilisateur participant dans le groupe de compétition à chaque utilisateur participant dans le groupe de compétition.

8. Procédé selon la revendication 6, dans lequel partager les informations de compétition extraites des informations de surveillance de santé en temps réel reçues de chaque utilisateur participant dans le groupe de compétition avec d'autres utilisateurs dans le groupe de compétition comprend en outre :
de fournir une comparaison finale des informations de compétition extraites des informations de surveillance de santé reçues de chaque utilisateur participant dans le groupe de compétition à chaque utilisateur participant dans le groupe de compétition.

9. Procédé selon la revendication 1, comprenant en outre :
de détecter une transmission des informations de surveillance de santé en temps réel depuis des terminaux utilisateur respectifs de deux ou plus ID utilisateur dans le groupe de réseau social ;
en réponse à la détection de la transmission, proactivement, sans requête des terminaux utilisateur respectifs des deux ou plus ID utilisateur dans le groupe de réseau social, de générer une invitation pour les deux ou plus ID utilisateur à participer à une compétition en temps réel basée sur une activité surveillée par les informations de surveillance de santé en temps réel ; et
d'envoyer l'invitation à chacun des deux ou plus ID utilisateur.

10. Serveur comprenant :
un ou plusieurs processeurs ;
de la mémoire ; et
un ou plusieurs modules de programme stockés dans la mémoire et configurés pour exécution par les un ou plusieurs processeurs, les un ou plusieurs modules de programme incluant :
un module de réception (160) configuré pour recevoir un premier identificateur de dispositif associé à un premier dispositif de surveillance de santé depuis un premier terminal utilisateur, le premier terminal utilisateur configuré pour communiquer avec le premier dispositif de surveillance de santé pour recevoir les informations de surveillance de santé d'un sujet surveillé respectif du premier dispositif de surveillance de santé ;
un module d'établissement (404) configuré pour établir une relation de correspondance entre le premier identificateur de dispositif et un premier ID utilisateur de la plate-forme de réseau social, dans lequel le premier ID utilisateur correspond à un premier utilisateur associé au premier terminal utilisateur ;
un module d'identification (406) configuré pour identifier au moins un second ID utilisateur dans une pluralité de contacts de réseaux sociaux du premier ID utilisateur en se basant sur le premier identificateur de dispositif, dans lequel chacun des au moins un second ID utilisateur est associé à un second identificateur de dispositif respectif pour un second dispositif de surveillance de santé qui fournit des informations de surveillance de santé d'un sujet surveillé respectif du second dispositif de surveillance de santé ; et
un module de formation (408) configuré pour former un groupe de réseau social respectif incluant le premier ID utilisateur et l'au moins un second ID utilisateur pour partager les informations de surveillance de santé reçues depuis le premier dispositif de surveillance de santé et le second dispositif de surveillance de santé respectif de l'au moins un second ID utilisateur ; **caractérisé en ce que** le module d'identification (406) comprend une première unité de détermination, une seconde unité de détermination, et une unité d'inclusion,
la première unité de détermination (501) est configurée pour déterminer un type de dispositif respectif pour le premier dispositif de surveillance de santé en se basant sur le premier identificateur de dispositif ;
pour chacun de la pluralité de contacts de réseaux sociaux du premier ID utilisateur :
la seconde unité de détermination (502) est configurée pour déterminer si le contact de réseau social est associé à un identificateur de dispositif respectif pour un dispositif du type de dispositif respectif ;
et
l'unité d'inclusion (503) est configurée pour inclure le contact de réseau social comme un des au moins un second ID utilisateur selon une détermination que le contact de réseau social et le premier ID utilisateur sont tous les deux associés aux identificateurs de dispositif respectifs du même type de dispositif.

11. Serveur selon la revendication 10, dans lequel :
le module de réception (160) est en outre configuré pour recevoir le premier identificateur de dispositif, le premier ID utilisateur, et des premières informations de surveillance de santé depuis le premier terminal utilisateur, dans lequel les premières informations de surveillance de santé sont collectées depuis le premier dispositif de surveillance de santé par le premier terminal utilisateur ;
le serveur comprend en outre un module de catégorisation (410) configuré pour catégoriser les premières informations de surveillance de santé en se basant sur au moins le premier identificateur de dispositif ou des paramètres extraits des premières informations de surveillance de santé ; et
le serveur comprend en outre un module de production (412) configuré pour produire une liste de partage d'informations de surveillance de santé incluant les premières informations de surveillance de santé reçues depuis le premier terminal utilisateur, dans lequel la liste de partage est accessible par le groupe de réseau social respectif.

12. Serveur selon la revendication 10, comprenant en outre un module de compétition en temps réel (414), dans lequel :
le module de compétition en temps réel (414) comprend une unité de réception, une unité de transfert, et une unité de formation,
l'unité de réception (402) est configurée pour recevoir une requête d'invitation du premier terminal utilisateur vers un ou plusieurs utilisateurs connectés dans le groupe de réseau social respectif pour une compétition en temps réel liée à une activité ; et
l'unité de transfert (505) est configurée pour envoyer une invitation respective à chacun des un ou plusieurs utilisateurs connectés selon la requête d'invitation, et
l'unité de formation (506) est configurée pour former un groupe de compétition incluant le premier ID utilisateur et un ou plusieurs utilisateurs connectés qui ont répondu à leurs invitations respectives.

13. Serveur selon la revendication 10, comprenant en outre un module d'invitation proactif (416), dans lequel :
le module d'invitation proactif (416) comprend une unité de détection, une unité de génération, et une unité de transfert,
l'unité de détection (507) est configurée pour détecter une transmission d'informations de surveillance de santé en temps réel depuis des terminaux utilisateur respectifs de deux ou plus ID utilisateur dans le groupe de réseau social respectif ; et
l'unité de génération (508) est configurée pour, proactivement en réponse à la détection de la transmission, sans requête des terminaux utilisateur respectifs des deux ou plus ID utilisateur dans le groupe de réseau social respectif, générer une invitation pour les deux ou plus ID utilisateur à participer à une compétition en temps réel en se basant sur une activité surveillée par les informations de contrôle de santé en temps réel ; et ;
l'unité de transfert (509) est configurée pour envoyer l'invitation à chacune des deux ou plus ID utilisateurs.
